(19) Europäisches Patentamt
European Patent Office
Office européen des brevets

(11) Numéro de publication : **0 362 001 B1**

(12) # FASCICULE DE BREVET EUROPEEN

(45) Date de publication du fascicule du brevet : **10.03.93 Bulletin 93/10**

(51) Int. Cl.$^5$ : **C07C 211/28,** C07C 255/42

(21) Numéro de dépôt : **89402366.2**

(22) Date de dépôt : **30.08.89**

(54) **N-cycloalkylalkyle benzylamines alpha,alpha disubstituées, leur procédé de préparation, leur utilisation comme médicament et leurs intermédiaires de synthèse.**

(30) Priorité : **01.09.88 FR 8811450**

(43) Date de publication de la demande : **04.04.90 Bulletin 90/14**

(45) Mention de la délivrance du brevet : **10.03.93 Bulletin 93/10**

(84) Etats contractants désignés : **AT BE CH DE ES FR GB GR IT LI LU NL SE**

(56) Documents cités :
**SYNTHESIS, vol. 2, fevrier 1979, pages 127-129; H. AHLBRECHT et al: "Dehydrocyanation of Alpha-Aminonitriles; A Versatile and Convenient Enamine and Dieneamine Synthesis"**
**JOURNAL OF THE CHEMICAL SOCIETY, Perkin Transactions I, 1980, pages 1450-1457; R. W. JEMISON et al; "Base Catalysed Rearrangements Involving Ylide Intermediates. Part 2. The Stevens (1,2) and (3,2) Sigmatropic Rearrangements of Allylic Ammonium Ylides"**
**JOURNAL OF THE CHEMICAL SOCIETY, Perkin Transactions I, 1980, pages 1458-1461; R. W. JEMISON et al: "Base Catalysed Rearrangements Involving Ylide Intermediates. Part 3. A Novel Thermal (1,3) Sigmatropic Rearrangement"**

(56) Documents cités :
**CHEMICAL ABSTRACTS, vol. 70, no. 9, 3 mars 1969, page 367, colonne 2, abrégé no. 37897u, Columbus, Ohio, USA; L. MIGINIAC et al; "Reaction of substituted alpha,beta-unsaturated organometallic compounds with aldimines"**
**CHEMICAL ABSTRACTS, vol. 79, no. 15, 15 octobre 1973, page 358, colonne 1, abrégé no. 91230v, Columbus, Ohio, USA; B. MAUZE et al: "Reversibility of the reaction between alpha-ethylenic organometallics and simple aldimines"**

(73) Titulaire : **JOUVEINAL S.A.**
**Tour Maine Montparnasse 33 Avenue du Maine**
**F-75755 Paris Cedex 15 (FR)**

(72) Inventeur : **Aubard, Gilbert**
**7 Chemin de la Savetière**
**F-91120 Palaiseau (FR)**
Inventeur : **Calvet, Alain**
**56 Avenue du Colonel de Rochebrune**
**F-92380 Garches (FR)**
Inventeur : **Gouret, Claude**
**34, rue Croix du Val**
**F-92190 Meudon (FR)**
Inventeur : **Grouhel, Agnès**
**2, rue des Peupliers**
**F-92190 Meudon (FR)**
Inventeur : **Jacobelli, Henri**
**65 Avenue du Général de Gaulle**
**F-91550 Paray Vieille Poste (FR)**
Inventeur : **Junien, Jean-Louis**
**36 Avenue Eiffel**
**F-92310 Sèvres (FR)**

(74) Mandataire : **Bourgognon, Jean-Marie et al**
**Cabinet Flechner 22, Avenue de Friedland**
**F-75008 Paris (FR)**

EP 0 362 001 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouvelles N-cycloalkylalkyle benzylamines disubstituées en position α, leur procédé de préparation et leur utilité sous forme de médicaments ainsi que leurs intermédiaires de synthèse.

Les benzylamines répondent à la formule générale I:

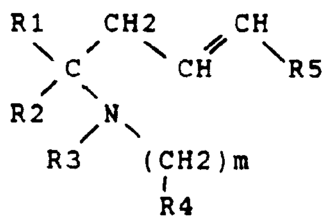

I

dans laquelle :

- R1 est phényle éventuellement mono, di ou trisubstitué par des halogènes, des radicaux alkyle inférieurs, haloalkyle ou alkoxy inférieurs,
- R2 est alkyle inférieur,
- R3 est hydrogène ou alkyle inférieur,
- m a pour valeur 1 ou 2,
- R4 est cycloalkyle -CH(CH2)n, dans lequel un atome de carbone peut porter un radical Rx qui est alkyle inférieur ou phényle ; et dans lequel n a les valeurs de 2 à 5.
- R5 est phényle pouvant être mono, di ou trisubstitué par des halogènes ou par des radicaux alkoxy inférieurs.

Dans cette description :

- par radical alkyle inférieur, on entend des radicaux linéaires ou ramifiés comprenant de 1 à 5 atomes de carbone,
- par halogène, on entend le brome, le fluor et le chlore à titre préféré,
- par haloalkyle et alkoxy inférieur, on entend respectivement de préférence les radicaux trifluorométhyl et méthoxy.

Les composés de l'invention (I) comprennent, adjacent à la fonction amine, un atome de carbone tetrasubstitué asymétrique qui leur donne la possibilité d'existence de ces composés sous formes racémique, lévogyre et dextrogyre, l'ensemble des isomères faisant partie intégrante de l'invention. De même, par la nature particulière des radicaux R1 à R5 ou par leur association, d'autres structures isomères sont possibles et font également partie de l'invention.

La fonction amine des composés I est appropriée à la préparation de sels d'addition avec les acides, sels dont l'hydrosolubilité potentielle est appréciée pour la préparation de certaines formes médicamenteuses.

Les sels de l'ensemble des composés I précédemment récapitulé avec les acides minéraux ou organiques thérapeutiquement acceptables sont également inclus dans l'invention, de même que leurs éventuels solvats.

A titre d'acides fréquemment utilisés pour la préparation des sels d'addition on cite de façon non limitative les acides acétique, benzènesulfonique, camphosulfonique, citrique, éthanesulfonique, fumarique, bromhydrique, chlorhydrique, lactique, maléïque, malique, méthanesulfonique, mucique, nitrique, pamoïque, phosphorique, salicylique, stéarique, succinique, sulfurique, tartrique.

Etudiées chez l'animal, les benzylamines (I) de l'invention et leurs sels se montrent faiblement toxiques et révèlent à la fois :

- une activité psychotrope montrée par leur aptitude à inhiber les crises convulsives provoquées par la picrotoxine,
- une activité à inhiber l'amnésie provoquée par administration de scopolamine,
- une activité gastro-duodénale par leur aptitude à inhiber l'activité ulcérogène de la cystéamine.

Aussi, les composés de l'invention sous forme de médicaments justifient d'une utilité indéniable et à ce titre les composés préférés sont ceux dans lesquels R1 est phényle, R2 est alkyle inférieur comprenant de 1 à 3 atomes de carbone et notamment est éthyle, R3 est méthyle, R4 est cycloalkyle CH(CH2)n non substitué et dans lequel n a pour valeur de 2 à 5 et R5 est phényle.

Plus particulièrement les composés préférés sont : l'α-cinnamyl-N-cyclohexylméthyl-α-éthyl-N-méthylbenzylamine, l'α-cinnamyl-N-cyclopropyléthyl-α-éthyl-N-méthyl-benzylamine, l'α-cinnamyl-N-cyclopropylméthyl-α-éthyl-N-méthyl-benzylamine et notamment pour les activités liées à l'affinité aux récepteurs sigma

son énantiomère dont le chlorhydrate est dextrogyre, et l'α-cinnamyl-N-cyclobutylméthyl-α-éthyl-N-méthylbenzylamine.

L'invention vise aussi un procédé de préparation des benzylamines (I) qui consiste :

- pour préparer un composé (II) de formule générale (I) et dans laquelle R3 est l'hydrogène et R1, R2, m, R4, R5 ont les significations précédemment énoncées,

i) à acyler une benzylamine (V) de formule :

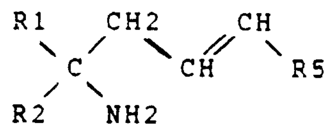

V

dans laquelle R1, R2 et R5 sont tels que précédemment définis, par un réactif (VI)

(R4-[CH2)m-1]-CO)pZ1 VI

dans lequel :

R4 et m ont les significations définies plus haut,

p a pour valeur 1 ou 2

Z1 est hydroxyle (-OH) ou halogène comme le chlore ou le brome lorsque p = 1 et est un atome d'oxygène lorsque p=2, pour obtenir un carboxamide intermédiaire (IV)

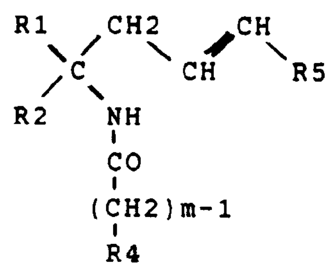

IV

que l'on réduit par un hydrure métallique en une benzylamine (II) de formule générale (I) dans laquelle R3 est l'hydrogène.

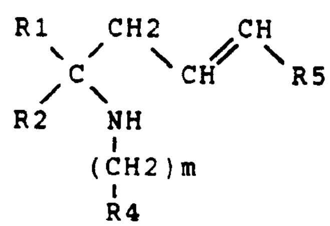

II

ii) à alkyler une benzylamine (V) par un halogénure d'alkyle R4-(CH2)m-Z2 dans lequel R4 et m ont les significations déjà citées et Z2 est un halogène tels que le chlore, le brome ou l'iode,

- et pour préparer une benzylamine (III) de formule générale (I) dans laquelle R3 est alkyle inférieur et R1, R2, m, R4, R5 ont les significations déjà précisées,

i) à procéder à une alkylation réductrice d'un composé (II) de l'invention qui consiste à faire réagir un aldéhyde R6-CHØ dans lequel R6 est l'homologue carboné immédiatement inférieur au radical R3 à introduire (R3=CH2-R6), puis un agent réducteur comme un hydrure métallique ou organométallique,

ii) ou à acyler une benzylamine (VII)

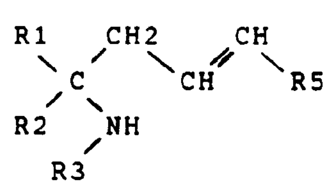

VII

dans laquelle R1, R2, R3 et R5 ont les significations précisées pour (I), par un halogénure R4-[(CH2)m-1]COZ4 dans lequel R4 et m ont les significations déjà définies et Z4 est un halogène et plus particulièrement le chlore ou le brome, pour obtenir le carboxamide intermédiaire (VIII)

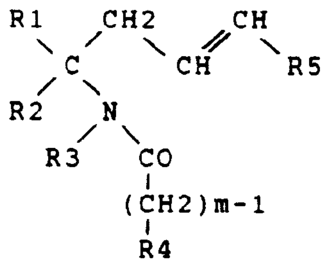

VIII

qui est réduit par un hydrure métallique en une benzylamine (III) de l'invention,

iii) ou, à faire réagir sur un intermédiaire amino nitrile (IX)

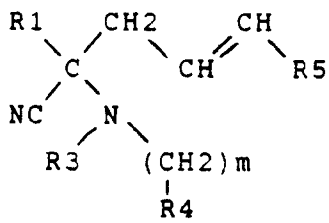

IX

dans lequel R1, R3, m, R4 et R5 sont tels que définis pour (I), un réactif organomagnésien R2MgZ3 dans lequel R2 est alkyle inférieur et Z3 halogène comme le chlore, le brome ou l'iode, pour obtenir une benzylamine (III).

iiii) ou encore à alkyler une benzylamine (VII) précédemment définie par un halogénure d'alkyle R4-(CH2)m-Z2 dans lequel R4 et m ont les significations définies pour (I) et Z2 est un halogène tel que le chlore le brome ou l'iode.

Telles que définies les benzylamines (I) de l'invention se différencient de l'art antérieur de par leur structure chimique et également de par leur application. Ainsi, L. Miginiac et B. Mauzé dans Bull. Soc. Chim. Fr., 1968, (9), p. 3832-44 puis Bull. Soc. Chim. Fr., 1973, (5) (Pt. 2), p. 1832-8 rapportent, au cours de l'étude de la réaction de dérivés organométalliques α-éthyléniques substitués sur des aldimines la préparation du N-méthylamino-1-diphényl-1,4 butène-3 de formule C6H5-CH-(NH-CH3)-CH2-CH=CH-C6H5 sans en indiquer d'application.

Par ailleurs, R.W. Jemison et coll. dans J. Chem. Soc., Perkin Trans. I., 198Ø, p. 145Ø-7 et 1458-61, au cours de l'étude de réarrangements de structures impliquant des intermédiaires de type "ylide" et catalysés par des bases, obtiennent et décrivent un produit f) à la page 1451 et à la page 1454, qui est le N,N-diméthylamino-1(p-nitrophényl)-1 phényl-4 butène-3 sans en indiquer d'application.

Ces produits diffèrent des benzylamines suivant l'invention par le fait que leur atome de carbone en position alpha dans la séquence benzylamine n'est que tri-substitué, alors que celui des benzylamines suivant l'invention porte, en plus, un radical alkyle. En outre, aucune activité pharmacologique susceptible d'application thérapeutique n'est rapportée pour ces produits.

Comme il a été précédemment exposé les composés intermédiaires qui permettent la préparation des produits (I) de l'invention sont essentiellement les dérivés de structure (V) (VII) et (IX).

Le procédé de préparation des composés (V) consiste à alkyler un composé (XVII) R1-CH2-W dans lequel R1 est tel que défini pour (I) et W est un radical nitrile (-CN) ou carboxyle (-COOH) par un halogènure d'alkyle de formule R2Z6, R2 étant défini pour (I) et Z6 étant un halogène, pour obtenir, pour W = -COOH un acide de formule (XV) R1(R2)-CH-COOH, et, pour W = -CN, obtenir un nitrile de formule (XVI) R1(R2)-CH-CN qui est hydrolysé en acide (XV), puis à alkyler l'acide (XV) par un halogènure d'alkènyle (XIII) de formule Z5-CH2-CH=CH-R5, Z5 étant un halogène et R5 tel que défini pour (I) pour obtenir les acides (XIV) R1(R2)C-(COOH)CH2- CH=CH-R5 puis à préparer par la réaction de Curtius à partir de ces acides les isocyanates (X).

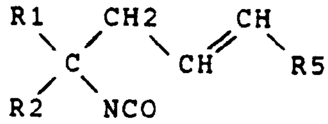

X

dans lesquels R1, R2 et R5 ont les significations énumérées pour (I), et finalement à hydrolyser leur fonction isocyanate pour obtenir les composés (V)

Plus précisément, la préparation d'un intermédiaire (V) consiste :

i) soit à monoalkyler par un halogènure d'alkyle R2-Z6 dans lequel Z6 est halogène, un acide phénylacétique R1-CH2-COOH (XVII) pour obtenir un acide (XV) : R1-(R2)CH-COOH puis à effectuer une seconde alkylation avec un halogénure d'alkényle (XIII) pour obtenir l'acide phénylacétique dialkylé (XIV) de formule :

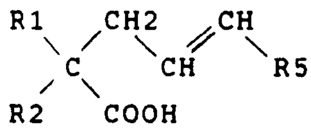

XIV

Les réactions d'alkylation sont réalisées par des méthodes connues comme celles présentées dans "Advanced Organic Chemistry" J. March, 3ème ed. (Wiley) p.421. qui consistent à alkyler les anions des acides obtenus par réaction de bases fortes sur les acides ou leurs sels.

Les bases fortes utilisées à cet effet peuvent être des dérivés métalliques ou organométalliques de métaux alcalins.

Ainsi, pour obtenir les acides (XV) et lorsque R1 ne comporte pas de substituant de nature halogènée comme des atomes de chlore ou des radicaux trifluorométhyl, à employer la méthode décrite dans "Journal of Organic Chemistry" 32, 9, p.2797-2803, 1967, qui consiste à préparer en solution dans le tetrahydrofurane le dianion de l'acide (XVII) par action de naphtalènate de sodium puis à faire réagir un halogènure qui est de préférence un dérivé iodé, pour obtenir l'acide (XV) puis, à réaliser la seconde alkylation de cet acide avec le dérivé (XIII) selon une méthode inspirée de celle décrite dans "Tetrahedron Lett." 198Ø, 21 (12) p.1169-72 qui consiste essentiellement pour former le dianion réactif à utiliser le diisopropylamide de lithium ( LDA ).

D'une manière plus précise, la première alkylation consiste à préparer d'abord le naphtalènate de sodium en milieu éthéré anhydre comme dans le THF par addition pour une mole de naphtalène en solution dans Ø,5 à 1 l. de THF, de Ø,9 à 1,1 mol de sodium puis à laisser la réaction se développer de 4 à 24 h. et plus favorablement de 12 à 18 h, à ajouter cette solution dans une autre solution de THF contenant de Ø,3 à Ø,5 mole d'acide (XVII) afin de former le dianion réactif par contact durant 1 à 24 h. à une température comprise entre 1Ø et 5Ø°C. Habituellement la réaction est complète entre 3 et 5 h. à 2Ø°C et il est introduit alors de Ø,3 à 1,2 mole de dérivé halogéné R2-Z6 et plus précisément, de Ø,45 à Ø,75 mole de ce dérivé ou l'halogène Z6 est l'iode. La réaction est complète après agitation entre 1 et 48 h. à une température comprise entre 1Ø et 5Ø°C. Plus favorablement le mélange est maintenu 16 à 2Ø h. à 2Ø-3Ø°C avant d'être traité pour obtenir le composé purifié (XV) attendu.

Ce composé est ensuite engagé dans la seconde réaction d'alkylation qui consiste à préparer "in situ" le LDA à partir de quantités équimoléculaires de diisopropylamine et de butyllithium puis, pour une mole de LDA ainsi préparé à ajouter dans le THF de Ø,5 à Ø,3 mole d'acide (XV) pour obtenir son dianion. Le dérivé halogéné (XIII) est ensuite introduit à une température comprise entre -1Ø et 5Ø°C puis le mélange laissé à réagir de 2 à 48 h. selon la réactivité des composés.

Ainsi, d'une manière préférée, à une mole de diisopropylamine dans 5ØØ ml de THF il est ajouté à -2Ø°C environ de Ø.95 à 1 mole de butyl lithium puis de Ø,4 à Ø,5 mole d'acide (XV) en solution dans environ 25Ø ml de THF. Après réaction de 1 à 2 heures entre 2Ø et 1ØØ°C pour former le dianion, le mélange est refroidi vers Ø°C et on y ajoute de Ø,4 à Ø,5 mole du dérivé halogéné (XIII).

La réaction est développée durant 1 à 2 h. à température ambiante puis le mélange est traité pour isoler et purifier le dérivé (XIV) obtenu.

ii) soit à monoalkyler par un halogènure d'alkyle R2-Z6 précédemment décrit un phénylacétonitrile R1-CH2-CN (XVII) pour obtenir un phénylacétonitrile alkylé (XVI) de formule R1(R2)-CH-CN dans lequel R1 et R2 ont les définitions décrites pour (I), Puis par réaction d'hydrolyse préparer l'acide (XV) qui est ensuite traité comme décrit précédemment pour obtenir l'acide (XIV). Cette préparation est préférée lorsque R1

est substitué par des atomes d'halogène en particulier de chlore ou par des radicaux haloalkyle inférieurs comme trifluorométhyle.

Pour ce faire, on utilise de préférence la méthode décrite dans "Il Farmaco" Ed. Sci. XXV (6) 197∅, p.4∅9-421 qui consiste à faire réagir un halogènure d'alkyle R2-Z6 sur un phénylacétonitrile (XVII) par une réaction utilisant un catalyseur dit de transfert de phase, en introduisant une mole d'acétonitrile dans une solution aqueuse de 2,5 à 3 moles de ce catalyseur comme le chlorure de benzyltriéthylammonium qui est préféré, puis ∅,75 à 1 mol de dérivé R2-Z6 dans lequel Z6 est le brome ou le chlore.

Après réaction de 1 à 48 h. et, plus habituellement de 3 à 5 h, le mélange est traité et le phénylacétonitrile monoalkylé est purifié, généralement par distillation sous pression réduite. Ce nitrile est hydrolysé, d'abord par l'acide bromhydrique en milieu éthanolique puis par une solution concentrée d'hydroxyde de sodium ainsi qu'il est décrit dans l'article cité, et ensuite à pratiquer la seconde alkylation telle que décrite en i) pour obtenir l'acide (XIV).

iii) puis préparer les isocyanates (X) selon la réaction de Curtius à partir des acides (XIV) préparés tel qu'il vient d'être décrit en i) et ii).

Des méthodes de réarrangement diverses (Hofman, Curtius et Lossen) permettent de préparer des isocyanates à partir de composés dérivés d'acides qui sont respectivement pour les réactions citées, les amides, les acides et les hydroxamates.

De façon préférée le procédé de préparation des intermédiaires (X) utilise la réaction de Curtius qui a été l'objet de publications indiquées par exemple dans la section "Organic Name Réactions" p.21 du "Merck Index" 1∅ème ed.. Elle consiste à partir d'un acide, à préparer successivement son chlorure puis l'azide correspondant et enfin par décomposition thermique de ce dernier à obtenir l'isocyanate souhaité.

La méthode avantageusement pratiquée permet de réaliser en une seule opération cette succession de réactions et consiste, dans un solvant halogéné apolaire, a faire réagir l'acide (XIV) avec de l'azide de sodium en présence de dichlorophosphate d'alkyle ou d'aryle comme les dichlorophosphate d'éthyle ou de phényle et d'une trialkylamine comme la triéthylamine ou d'une amine aromatique comme la pyridine qui est préférée, puis à éliminer le solvant et procéder directement au réarrangement de l'azide formé par action de la chaleur.

Pratiquement, pour 1 mole d'acide en solution dans 3 à 2∅ litres de dichlorométhane on ajoute de 1 à 1,75 mol de dichlorophosphate de phényle puis de 2 à 3,5 mol d'azide de sodium et de pyridine en quantités équimoléculaires. La formation de l'azide est réalisée à une température comprise entre 1∅ et 4∅°C en agitant de 4 à 24 h selon la réactivité des produits.

Après traitements à l'eau et à l'acide chlorhydrique le dichlorométhane est éliminé par distillation tout en ajoutant un solvant inerte de point d'ébullition supérieur à 1∅∅°C apte à être utilisé comme solvant de la réaction de décomposition thermique de l'acide en isocyanate.

Plus favorablement, on utilise un solvant aromatique comme le toluène et la réaction de décomposition est effectuée à la température du reflux de ce solvant jusqu'à fin de dégagement gazeux ce qui nécessite une durée comprise entre 3∅ minutes et 8 heures.

L'isocyanate (X) est obtenu après évaporation du solvant puis éventuellement purifié et,

iiii) à finalement hydrolyser l'isocyanate (X) pour obtenir les composés intermédiaires de l'invention de formule (V).

D'une manière générale, cette hydrolyse est catalysée par les acides ou les bases de préférence inorganiques tels que les acides bromhydrique, sulphurique, phosphorique et chlorhydrique qui est l'acide préféré ou les hydroxydes de métaux alcalins ou alcalino-terreux, les hydroxydes de sodium et de potassium étant préférés. L'hydrolyse peut être pratiquée en milieu aqueux ou en présence d'un solvant miscible à l'eau et non réactif avec les composés de la réaction. Les éthers comme les dioxanes et plus particulièrement le tétrahydrofurane (THF) sont préférés en mélange avec l'eau.

Ainsi pour une mole de dérivé (X) à hydrolyser la réaction est pratiquée en dissolvant le produit dans ∅,5 à 1∅ litres de THF puis de l'eau est ajoutée en proportions variables selon le dérivé à hydrolyser, la composition relative du mélange THF-eau (v/v) pouvant varier dans des proportions comprises entre 5-95 et 95-5.

Le catalyseur acide, par exemple l'acide chlorhydrique sous forme de solution aqueuse concentrée, est ajoutée à raison de ∅,2 à 1∅,∅ mol par mole de composé (X) et plus généralement à raison de ∅,5 à 5 mol.

Le milieu réactionnel est ensuite porté à une température comprise entre 5∅°C et la température de reflux des solvants, à laquelle il est maintenu de 2 à 72 h pour obtenir une quantité satisfaisante de produit.

Habituellement, une durée de chauffage de 5 à 24 heures est nécessaire après quoi le solvant est éliminé par distillation, le résidu aqueux traité pour isoler l'amine primaire de formule (V) formée et le produit finalement purifié par distillation, cristallisation ou chromatographie comme il est précisé dans la partie expérimentale du texte.

Le procédé de préparation des composés (VII) consiste :

i) pour obtenir plus particulièrement un composé dans lequel R3 est méthyle, à réduire l'isocyanate intermédiaire (X) précédemment décrit. Il est utilisé comme agent réducteur un hydrure métallique ou organométallique dans des conditions appropriées à la réduction spécifique de la fonction isocyanate sans agir sur la liaison éthylènique des composés.

A cet effet, on utilise favorablement l'hydrure de lithium aluminium ou l'hydrure d'aluminium qui est préféré. Les réactions sont effectuées dans des solvants inertes aux réactifs utilisés tels que dans des éthers comme par exemple l'éther diéthylique, le 1,2-diméthoxy éthane ou le tétrahydrofuranne (THF) qui est préféré.

Avantageusement, l'agent réducteur utilisé, l'hydrure d'aluminium peut être préparé "in situ" à partir d'halogènures d'aluminium et d'hydrures métalliques comme il est par exemple décrit dans "Réduction with complex métal hydrides" - N.G. Gaylord., 1956, Ed. Interscience - p. 6 à 8, 51 à 53.

Ainsi, la réaction de réduction dans le THF d'une mole d'isocyanate (X) consiste dans un premier temps à préparer "in situ" l'hydrure d'aluminium par réaction sur Ø.75 à 2 moles de chlorure d'aluminium, de 2,25 à 6 moles d'hydrure de lithium aluminium, ces réactifs étant utilisés dans un rapport moléculaire voisin de 1 pour 3, puis à introduire à une température comprise entre - 1Ø et + 3Ø°C l'isocyanate, à laisser la réaction de réduction se développer de 1 à 24 h à la même température, puis à décomposer le complexe réduit obtenu et à isoler la N-méthyl amine de formule (VII) par les méthodes habituelles.

Couramment, ces réductions sont effectuées à une température comprise entre 1Ø et 2Ø°C durant 2 à 6 h.

ii) pour obtenir une benzylamine (VII) dans laquelle R3 est indifféremment alkyle inférieur tel que décrit pour (I) à acyler un intermédiaire (V) par un réactif (R7-CO) pZ4 dans lequel R7 est hydrogène ou un radical alkyle inférieur homologue inférieur à R3 et Z4 est halogène comme le chlore ou le brome ou est encore hydroxyle quand p est égal à 1 ou Z4 représente encore l'oxygène quand p est égal à 2, pour obtenir un intermédiaire (XI)

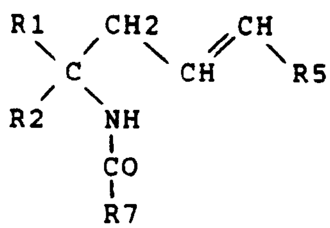

XI

dont la fonction N-carboxamide est réduite par un hydrure métallique.

iii) ou, d'une façon alternative, à alkyler un intermédiaire (V) par un halogènure d'alkyle R3-Z6 dans lequel Z6 représente le chlore, le brome ou l'iode.

Le procédé de préparation des amino nitriles (IX) consiste à préparer un amino nitrile (XII)R1(NC)CH-N(R3)R4 à partir d'une benzaldéhyde R1-CHØ, d'une amine secondaire HN(R3)R4 et d'un cyanure de métal alcalin selon la réaction de Strecker et par une technique décrite par S.F. Dyke et coll., Tetrahedron 1975, 31, p. 1219, puis à alkyler le dérivé (XII) par un halogénure de cinnamyle Z5-CH2-CH=CH-R5 (XIII) dans lequel R5 a les valeurs définies pour (I) et Z5 est le chlore, le brome ou l'iode.

Cette alkylation est réalisée en préparant d'abord l'anion de l'amino nitrile (XII) par action dans un solvant inerte comme le THF d'une base organométallique forte appropriée. Le N,N diisopropyl amidure de lithium est préféré. Il est préparé "in situ" à partir de quantités équimoléculaires de diisopropylamine et de butyllithium.

Après réaction du composé (XII) durant 1 à 2 h entre 2Ø et 1ØØ°C on ajoute à l'anion l'halogénure de cinnamyle (XIII) et laisse la réaction se développer de 1 à 2 h à la température ambiante pour obtenir l'intermédiaire (IX) qui est purifié.

D'une manière plus explicite que celle précédemment présentée la préparation de composés de l'invention (II) et (III) appartenant à la formule générale (I) est développée dans ce qui suit :

a) lorsque le procédé consiste à acyler un composé (V) par un réactif (VI) pour obtenir un intermédiaire N-carboxamide (IV) qui est ensuite réduit.

Lorsque le réactif (VI) est un halogénure d'acide (p = 1, Z1 = halogène) la réaction préférée s'effectue en milieu monophasique dans le toluène ou plus favorablement le dichlorométhane, et consiste à ajouter dans une solution contenant une mole de dérivé à acyler de 1,Ø à 1,5 mol d'amine qui est généralement la triéthylamine, puis à ajouter le réactif (VI) en quantité équimoléculaire à la triéthylamine. La solution est ensuite maintenue de 3 à 48 h à une température comprise entre 15 à 3Ø°C de façon à obtenir la réaction la plus complète possible.

Et lorsque le réactif d'acylation (VI) est un anhydride d'acide (p = 2 ; ; Z1 = oxygène) la réaction, lorsque le point d'ébullition de l'anhydride est inférieur à 14Ø°C peut s'effectuer sans solvant, en faisant réagir le composé (V) dans un large excès et à la température de reflux du réactif (VI). La méthode préférée consiste cependant à réaliser la réaction en utilisant comme solvant la pyridine et en faisant réagir pour une mole de composé à acyler, de 1 à 5 mole d'anhydride. Couramment l'utilisation de 1,2 à 1,8 mole d'anhydride au reflux de la pyridine durant 1 à 3 heures conduit à des résultats convenables.

La méthode d'acylation préférée de (V) lorsque le réactif (VI) est un acide carboxylique (p = 1 ; m = 1 ou 2 ; Z1 = OH) consiste à préparer, in situ, un anhydride pouvant être mixte comprenant l'acide carboxylique puis à acyler l'intermédiaire (V) par cet anhydride.

Favorablement, la réaction est effectuée dans des solvants apolaires anhydres de la classe des éthers oxydes. Le tetrahydrofurane est préféré et on forme d'abord l'anhydride mixte à une température comprise entre -4Ø et Ø°C en ajoutant pour une mole d'acide (VI) de 1,Ø à 1,5 mole d'amine tertiaire comme la N-méthylmorpholine puis de Ø,9 à 1,2 mole de chloroformiate d'isobutyle.

On ajoute ensuite une mole d'intermédiaire (V) à acyler et laisse la réaction se développer de 1 à 48 heures à une température comprise entre Ø et 6Ø°C.

Habituellement, le résultat de la réaction est satisfaisant à une température comprise entre 1Ø et 25°C après une durée de 1Ø à 2Ø heures.

Des méthodes alternatives peuvent employer d'autres agents de déshydratation énumérés par exemple dans "Advanced Organic Chemistry", J. March. Ed. Wiley 1985. p. 349. Ainsi la réaction a été réalisée avec pour agent de déshydratation en milieu anhydre le dicyclohexylcarbodiimide et plus particulièrement avec l'acide formique, et en utilisant le N-N'-carbonyldiimidazole.

La réduction des intermédiaires N-carboxamides est réalisée par des hydrures métalliques ou organométalliques de façon appropriée à la réduction spécifique de la fonction carboxamide sans agir sur la liaison éthylénique des composés.

A cet effet, on utilise favorablement l'hydrure de lithium aluminium ou l'hydrure d'aluminium qui est préféré. Les réactions sont effectuées dans des solvants inertes aux réactifs utilisés tels que dans des éthers comme par exemple l'éther diéthylique, le 1,2-diméthoxy éthane ou le tétrahydrofurane (THF) qui est préféré.

Egalement de façon préférée, l'agent réducteur utilisé, l'hydrure d'aluminium peut être préparé "in situ" à partir d'halogènures d'aluminium et d'hydrures métalliques comme il est par exemple décrit dans "Réduction with complex métal hydrides" - N.G. Gaylord., 1956, Ed. Interscience - p. 6 à 8, 51 à 53.

Avantageusement, la réaction de réduction dans le THF d'une mole d'intermédiaire (IV) ou (VIII) consiste dans un premier temps à préparer "in situ" l'hydrure d'aluminium par réaction sur Ø.75 à 2 moles de chlorure d'aluminium, de 2,25 à 6 moles d'hydrure de lithium aluminium, ces réactifs étant utilisés dans un rapport moléculaire voisin de 1 pour 3, puis à introduire à une température comprise entre - 1Ø et + 3Ø°C l'intermédiaire N-carboxamide à laisser la réaction de réduction se développer de 1 à 24 h à la même température, puis à décomposer le complexe réduit obtenu et à isoler les composés de l'invention (II) ou (III) par les méthodes habituelles.

Le plus couramment, les réductions sont effectuées à une température comprise entre 1Ø et 2Ø°C durant 2 à 6 h.

Tel qu'il a été précédemment décrit pour l'utilisation du réactif (VI) lorsqu'il est un halogènure d'acide, la réaction s'applique également à l'acylation des intermédiaires (VII) par les réactifs R4-[(CH2)m-1]-CØ-Z4 dans lesquels Z4 est le chlore ou le brome, afin de préparer les carboxamides intermédiaires (VIII) qui, réduits tel qu'indiqué, permettent d'obtenir les benzylamines (III) de l'invention.

b) Lorsque le procédé consiste à N-alkyler un intermédiaire (V) ou (VII) par un halogénure d'alkyle R4-(CH2)m-Z2 déjà décrit et dans lequel Z2 est le chlore, le brome ou l'iode la réaction est réalisée dans des solvants inertes aux réactifs comme par exemple le toluène et l'acétonitrile, et en faisant réagir une mole d'intermédiaire (V) ou (VII) avec de Ø,5 à 1,5 mol d'halogénure.

D'une manière préférée, on utilise de Ø,8Ø à 1,2Ø mol de dérivé ou l'halogène est le brome ou l'iode et optionnellement on ajoute une base, organique ou minérale, pour favoriser la réaction qui consiste à chauffer le milieu réactionnel à une température comprise entre 2Ø et 11Ø°C et ce durant de 2 à 5 h, les produits étant ensuite isolés et purifiés par les méthodes habituelles, notamment par chromatographie,

c) lorsque le procédé consiste à pratiquer une N-alkylation réductrice pour obtenir un composé de l'invention (III) à partir d'un composé (II) et d'un aldéhyde R6-CHØ il peut être pratiqué différentes techniques dont l'essentiel est présenté dans "Advanced Organic Chemistry", J. March - 3ème Ed. - Wiley 1985 - p. 798-8ØØ.

Avantageusement, pour les divers réactifs carbonylés employés, excepté le formaldéhyde, la réaction peut être effectuée dans un solvant protique anhydre comme les alcools inférieurs tels que le méthanol, l'éthanol, en faisant réagir un mole de composé II avec 1,5 à 1Ø mole de composé carbonylé en présence d'un catalyseur acide anhydre comme l'acide acétique, l'acide p. toluéne-sulfonique.

La réaction est ainsi réalisée entre 3Ø minutes et 8 heures à une température comprise entre celle des

laboratoires et celle de reflux du solvant. Puis à température ambiante on ajoute un hydrure réducteur de bore comme le borohydrure de sodium ou le cyanoborohydrure de sodium à raison de Ø,5 à 2,5 mole par mole de composé (II) engagé.

Particulièrement, quand le procédé consiste en l'alkylation d'un composé (II) par le formaldéhyde pour obtenir un produit de l'invention (III) dans lequel R3 est méthyle, on pratique avantageusement la méthode décrite dans J. Med. Chem. 1982, 25, 4, p. 446-51, qui consiste à faire réagir dans l'acétonitrile, le formaldéhyde en solution aqueuse en présence de cyanoborohydrure de sodium.

d) Lorsque le procédé pour préparer un composé de l'invention (III) consiste à faire réagir un aminonitrile (IX) avec un réactif organomagnésien R2MgZ3, la substitution de la fonction nitrile par le radical R2 est réalisée selon une méthode inspirée de celle décrite par N.J. Léonard et coll., J. Am. Chem. Soc., 1956, 78, p. 1986 et 1957, 79, p. 5279. Elle est effectuée dans les éthers comme l'éther diethylique, le méthyl-t.butyl ether, les ethers di-isopropylique ou dibutylique ou encore le tétrahydrofurane qui est préféré, et consiste pour une mole de composé (IX) à faire réagir 1,5 à 6 moles de dérivé organo magnésien à une température comprise entre 5 et 5Ø°C et ce durant 3Ø minutes à 12 heures.

Avantageusement, la méthode consiste à ajouter à une température comprise entre 1Ø et 2Ø°C 1 mol de composé (IX), éventuellement en solution dans le THF à 4 à 5 moles du composé organomagnésien également en solution dans le THF. La réaction est poursuivie durant de 2 à 5 heures à la même température puis le complexe obtenu décomposé par addition de solution aqueuse de chlorure d'ammonium. Après traitements, le composé de l'invention (III) est isolé et purifié.

Telle que définie, l'invention comprend les composés de formule générale (I) sous leur forme racémique et leurs formes optiquement actives.

La préparation de stéréoisomères est réalisée :

- soit par résolution des racémiques des composés (II) ou (III)
- soit à partir de précurseurs optiquement actifs, notamment les formes énantiomères (V), eux-mêmes préparés par résolution de leurs racémiques.

Les méthodes de résolution sont diverses et répertoriées dans des ouvrages de la littérature scientifique comme "Optical résolution procédures for Chemical Compounds" Vol. 1 - Amines and related compounds - Ed. Paul Newman 1981.

Il est proposé de nombreux énantiomères d'acides qui peuvent permettre de réaliser ces résolutions à partir des produits racémiques de l'invention de structure (II), (III) ou de leurs intermédiaires (V).

Avantageusement, le procédé de l'invention consiste à former dans l'eau des diastéréoisomères de l'acide tartrique lévogyre ou de l'acide tartrique dextrogyre avec les produits racémiques de formule (V) et généralement, dans les conditions de la séparation, à précipiter un sel constitué par un des énantiomère de (V) qui a une rotation opposée à celle de l'acide tartrique utilisé.

La purification des produits est réalisée par cristallisations répétées jusqu'à l'obtention d'une valeur de pouvoir rotatoire constante.

Les modes opératoires qui suivent illustrent de façon non limitative la préparation de dérivés intermédiaires et des composés (I) de l'invention représentés par les produits de formules (II) et (III).

Les composés sont, selon les réactions effectuées, soit obtenus tels quels dans un état de pureté satisfaisant, soit purifiés par des techniques appropriées indiquées dans les exemples comme la cristallisation, la distillation sous vide ou encore la chromatographie sur colonne. Dans ce dernier cas on utilise favorablement la technique dite de "chromatoflash" sur un support de silice (marque "Merck", produit Kieselgel 6Ø, granulométrie 23Ø à 4ØØ mesh).

Par ailleurs, la pureté, l'identité et les caractéristiques physico chimiques des produits préparés sont rapportées et déterminées par :

- leur point d'ébullition sous la valeur du vide lors de leur distillation,
- leur point de fusion, déterminé par la méthode du tube capillaire et dont la valeur indiquée n'est pas corrigée,
- la chromatographie sur couches minces (CCM) de silice (plaques prêtes à l'emploi, "Merck" ref. 6Ø F 254) dont la technique est brièvement rappelée :

les produits à étudier sont déposés sur la plaque à raison de 1ØØ mcg environ puis élués de façon ascendante par des solvants ou leurs mélanges qui sont énumérés ci-après, les proportions respectives étant indiquées en volumes pour volumes :

| | | | | | | |
|---|---|---|---|---|---|---|
| réf. S.A | - | hexanes | 100 / | acétate d'éthyle | 10 |
| S.B | - | " | 60 / | " | 10 |
| S.C | - | " | 40 / | " | 10 |
| S.D | - | " | 20 / | " | 10 |
| S.E | - | " | 10 / | " | 10 |
| S.F | - | dichlorométhane | 20 / | hexanes | 80 |
| S.G | - | dichlorométhane | | | |
| S.H | - | " | 90 / | acétone | 10 |
| S.I | - | " | 85 / | " | 15 |
| S.J | - | " | 80 / | " | 20 |
| S.K | - | " | 98 / | méthanol | 2 |
| S.L | - | " | 95 / | " | 5 |
| S.M | - | " | 90 / | " | 10 |
| S.N | - | " | 85 / | " | 15 |

Après développement, les chromatogrammes sont observés sous lumière ultra violette de 254 nm de longueur d'onde et/ou après révélation colorée par pulvérisation du réactif de Dragendorff ou du réactif à la tolidine. Les Rf observés ainsi que les références des solvants d'élution utilisés sont indiqués dans les exemples.

- l'analyse centésimale élémentaire dont les résultats, conformes aux normes admises ne sont pas reportés, sont signalés être effectués par la représentation de l'élément dosé,
- la résonance magnétique nucléaire du proton (RMN) est étudiée à 60 ou 90 MHz, les produits étant solubilisés dans le deutérochloroforme. L'aspect des signaux, leur déplacement chimique exprimé en p.p.m par rapport au tétraméthylsilane utilisé comme référence interne sont indiqués. Les protons dits "échangeables" après addition d'oxyde de deutérium sont également signalés.
- La mesure du pouvoir rotatoire est exprimé par la rotation optique spécifique de produits ($\alpha$) dans des conditions (concentration, solvant) indiquées de façon conventionnelle.

Enfin, divers réactifs ou solvants peuvent être indiqués sous leur forme abrégée courante, entre autres exemples, THF pour le tétrahydrofurane.

## PARTIE EXPERIMENTALE

### Préparation d'intermédiaires

### A. Intermédiaires de formule (XV)

#### A.1 / Acide 2-p.méthoxyphényl-butanoïque.

(R1 = p.CH30-C6H4 ; R2 = C2H5)

Dans un réacteur protégé de l'humidité et sous atmosphère d'azote, on introduit 29,4 g (0,23 mol) de naphtalène dans 200 ml de THF. A cette solution il est ajouté 5,5 g (0,23 mol) de sodium en morceaux préalablement dégraissés par du toluène. On obtient une solution verdâtre qui est maintenue sous agitation durant une nuit.

Par ailleurs dans un autre réacteur, 16,6 g, (0,10 mol) d'acide p.méthoxyphénylacétique sont dissous dans 200 ml de THF. La solution de naphtalènate de sodium précédemment préparée est introduite sous agitation, le mélange est maintenu 4 heures à température ambiante puis on ajoute en une heure environ 23,4 g (0,15 mol) d'iodoéthane.

Après une nuit d'agitation la suspension est précipitée dans 150 ml de solution de carbonate de sodium à 10 % p/v. La phase aqueuse est extraite à l'éther, les phases organiques réunies lavées par une solution HCl N puis avec une solution saturée en NaCl.

L'ether est évaporé, le résidu cristallisé dans 150 ml d'éther de pétrole.

Poids = 16,1 g Rdt = 83% F = 64°C

Les intermédiaires acides A.2, 3 et 4 préparés selon le mode opératoire précédent à partir des acides phénylacétiques substitués et des halogènures d'alkyle appropriés.

**A.2 / Acide 3 - méthyl-2-phényl butanoïque.**

(R1 = C6H5 ; R2 = (CH3)2-CH)
Rdt = 67% F = 70°C

**A.3 / Acide 2 - p. chlorophényl-butanoïque.**

(R1 = p.Cl-C6H4 ; R2 = C2H5)

Dans un réacteur on introduit à 5°C environ et sous agitation violente 81,4 g (0,537 mol) de p.chlorophénylacétonitrile dans une solution de 59,2 g (1,48 mol) de soude en pastilles dans 60 ml d'eau, puis on ajoute 1,2 g (52 mmol) de chlorure de benzyltriéthyl- ammonium. Le mélange est maintenu 10 minutes à 5°C puis après retour à la température ambiante, 50,3 g (0,462 mol) de bromure d'éthyle sont introduits en 40 minutes environ et à 20°C.

Le mélange de couleur rouge est agité 4 h puis abandonné une nuit à 4°C.

Après addition de 560 ml d'eau, on extrait au benzène. Les phases organiques réunies sont lavées par une solution saturée en chlorure de sodium. Le benzène est évaporé, le nitrile (XVI) obtenu est purifié par distillation :

Eb/0,05 = 85-95°C
Poids = 84,0 g Rdt = 87%

Sous atmosphère anhydre, 0,47 mol du nitrile et 21,5 ml d'éthanol absolu sont saturés à -15°C par un courant d'acide bromhydrique gazeux. Après une nuit à la température ambiante on ajoute 880 ml d'acétone puis chauffe et porte une heure au reflux. La solution est concentrée au bain marie et sous vide puis on ajoute au résidu huileux 800 ml de solution concentrée d'hydroxyde de sodium à 30 % p/v et agite au reflux et sous agitation pendant 30 heures.

Le mélange est refroidi, extrait au chloroforme, la phase aqueuse alcaline est acidifiée à froid jusqu'à pH 1 par une solution concentrée d'acide sulfurique diluée au demi.

Après extraction au chloroforme et traitements habituels des phases organiques, les solvants sont évaporés et le résidu obtenu cristallisé pour purification dans 300 ml d'éther de pétrole.
Poids = 60,5 g Rdt = 65 % F = 84°C

**A.4 / Acide 2 - (m.trifluorométhyl) phényl-butanoïque.**

(R1 = m.F3C-C6H4 ; R2 = C2H5)

Le composé est préparé selon le mode opératoire de l'exemple précédent à partir de m. trifluorométhylphénylacétonitrile. On obtient les produits suivants :

| | | | |
|---|---|---|---|
| nitrile | XVI | : Rdt = 58% | Eb/0,06 = 75 - 80°C |
| acide | XV | : Rdt = 77% | F = 72°C |

**B - Intermédiaires de formule (XIV)**

**B.1 / Acide α-cinnamyl-α-éthyl-phénylacétique.**

(R1 = R5 = C6H5 ; R2 = C2H5)

Une solution de 339 g de diisopropylamine (3,35 mol) dans 2,2 l de THF anhydre, dans un réacteur de 12 litres protégé de l'humidité et sous atmosphère d'azote, est refroidie à -10°C. On ajoute 330 ml de solution de butyl-lithium 10M dans l'hexane (3,30 mol), lentement et à une température inférieure à -10°C. La solution est maintenue 30 minutes à cette température puis on ajoute une solution de 250 g (1,52 mol) d'acide 2 - phényl butanoïque dans 300 ml de THF en laissant la température remonter progressivement jusqu'à 15°C. On chauffe ensuite à 55 - 60°C durant 3 h, refroidit à 5°C et introduit sans dépasser 15°C 300 g (1,52 mol) de bromure de cinnamyle en solution dans 250 ml de THF.

On maintient sous agitation à la température du laboratoire durant 18 h puis sans dépasser 30°C ajoute 2 l de solution HCl 3N puis 1 l d'eau ; on extrait par 2 fois 1,5 l d'acétate d'éthyle, ajoute 4 l d'hexanes aux

phases d'extractions réunies, lave le mélange par 2 fois 1,2 l de solution NaOH N. Les phases aqueuses alcalines sont acidifiées par une solution d'HCl concentré puis extraites par 2 fois 1,5 l d'acétate d'éthyle. Les phases organiques réunies sont lavées par une solution saturée en chlorure de sodium puis concentrées par distillation.

Le produit brut résiduel obtenu (428 g) est purifié par cristallisation dans 2 l de mélange hexanes - acétate d'éthyle 3/1 v/v. Le produit est obtenu sous forme de fins cristaux blancs.

| | |
|---|---|
| Poids = 320 g | Rdt = 75% |
| F = 134 - 136°C | CCM : 0,40 ; S.C |

L'alkylation de l'acide α-méthyl-phénylacétique et de l'acide 3-méthyl-2-phényl butanoïque (préparation A.2) par le bromure de cinnamyle est réalisée selon le mode opératoire de l'exemple précédent et conduit aux acides (XIV) B.2 et B.3.

**B.2 / Acide α-cinnamyl-α-méthyl phénylacétique**

(R1 = R5 = C6H5 ; R2 = CH3)
Rdt = 83 %    F = 135°C (hexanes)    CCM: 0,30 ; S.H

**B.3 / Acide α-cinnamyl-α-isopropyl-phénylacétique.**

(R1 = R5 = C6H5 ; R2 = CH3)
Rdt = 55 %    F = 97°C (hexanes)    CCM: 0,70 ; S.H

Le procédé de l'exemple B.1 appliqué au 1-(3-bromo-1-propènyl)-3,4-dichlorobenzène (intermédiaire XIII décrit en C.1) avec les acides intermédiaires (XV) décrits en A.1, A.3 et A.4.permet d'obtenir les composés B4 à B6

**B.4 / Acide α-(3',4'-dichloro)cinnamyl-α-éthyl-p.méthoxyphénylacétique.**

(R1 = p.CH3O-C6H4 ; R2 = C2H5 ; R5 = 3,4 Cl2-C6H3)
Rdt = 70%    F = 135°C (éth. pétr.)    CCM: 0,60 ; S.J

**B.5 / Acide α-(3',4'-dichloro)cinnamyl-α-éthyl p.chloro phénylacétique.**

(R1 = p.Cl-C6H4 ; R2 = C2H5 ; R5 = 3,4 Cl2-C6H3)
Rdt = 70 %    F = 160°C (éth. pétr.)    CCM: 0,60 ; S.L

**B.6 / Acide α-(3',4'-dichloro)cinnamyl-α-éthyl-(3-trifluorométhyl)phénylacétique.**

(R1 = m.F3C-C6H4 ; R2 = C2H5, ; R5 = 3,4 Cl2-C6H3)
Rdt = 62 %    F = 96°C (hexanes)    CCM: 0,40 ; S.L

**C - Intermédiaires de formule (XIII)**

**C.1 1 - (3-bromo-1-propènyl)-3,4-dichlorobenzène.**

(R5 = 3,4 Cl2-C6H3 ; Z5 = Br)

Dans un réacteur protégé de l'humidité et sous atmosphère d'azote 100,6 g (0,46 mol) d'acide 3,4-dichloro cinnamique sont mis en suspension dans 1700 ml de méthanol.

Après addition de 56,6 ml de complexe BF3 - éther (65,3 g, 0,46 mol) le mélange est porté au reflux sous agitation pendant 18 h. La solution est évaporée, le résidu repris par environ 1 l. de dichlorométhane, la solution obtenue lavée avec une solution saturée en bicarbonate de sodium puis à l'eau. Après distillation le résidu est purifié par cristallisation dans l'éthanol. On obtient 99,2 g de 3,4- dichlorocinnamate de méthyle sous forme de cristaux blancs.

Rdt = 93%    F = 115°C

Sous atmosphère d'azote, il est ajouté à une solution de 5,0 g (0,24 mol) de l'ester précédent dans 540 ml de toluène, à -40°C et en une heure environ, 300 g d'une solution toluénique 1,5 M de DIBAL-H(R). Le mélange est maintenu 2 h 3Ø à -4Ø°C puis après retour à une température de 1Ø°C environ, on introduit avec précautions 1 litre de solution d'acide sulfurique environ 2 M. La phase toluénique est séparée, la phase acide extraite à l'éther. Les phases organiques réunies sont lavées par une solution saturée en bicarbonate de sodium puis séchées sur Na2SO4.

Les solvants sont éliminés par distillation et le résidu purifié par chromatographie sur colonne.

L'élution par le mélange dichlorométhane-acétone 8Ø/2Ø puis une cristallisation dans l'hexane permet d'obtenir 47,9 g (Rdt = 97 %) d'alcool 3,4-dichlorocinnamique purifié F = 64°C.

Dans un réacteur protégé de l'humidité et sous atmosphère d'azote, on introduit une solution de 25,1 g (Ø,29 mol) de bromure de lithium dans 225 ml d'acétonitrile.

Puis, à 4Ø°C sous agitation, on ajoute successivement, en 3Ø minutes, 39,3 g (Ø,36 mol) de chlorotriméthylsilane et ensuite en 2Ø minutes, 29,45 g (Ø,145 mol) d'alcool 3,4-dichlorocinnamique en solution dans 125 ml d'acétonitrile.

Le mélange, sous agitation, est maintenu au reflux 16 heures puis refroidi et précipité dans 4ØØ ml d'éther et 25Ø ml d'eau glacée. La phase organique séparée est lavée par une solution saturée en bicarbonate de sodium puis avec une solution saturée en NaCl. Les solvants sont éliminés et le produit brut résiduel purifié par distillation.

Poids = 35,1 g      Rdt = 91 %      Eb/Ø,1 = 115-12Ø°C.

**C.2 / 1-(3-chloro 1-propènyl)- 3,4,5 triméthoxybenzène.**

(R5 = 3,4,5(CH3O)3-C6H2 ; Z5 = Cl)

A une température de -1Ø°C et sous atmosphère d'azote, il est introduit lentement dans une suspension de 25,7 g (Ø,193 mol) de chlorure d'aluminium dans 3ØØ ml d'éther, une suspension de 22,Ø g (Ø,58 mol) d'hydrure de lithium-aluminium dans 3ØØ ml de THF. A la même température, en solution dans 28Ø ml de THF, 73,Ø g (Ø,29Ø mol) de 3-(3,4,5-triméthoxyphényl)-2-propènoate de méthyle sont introduits en 1Ø minutes. Le mélange est agité ensuite 15 minutes puis précipité dans une solution glacée d'acide sulfurique 2,5 M, extrait à l'éther. Les phases organiques réunies sont lavées, séchées. L'évaporation des solvants conduit à l'alcool 3,4,5-triméthoxycinnamique qui est utilisé tel quel.

Une solution de 62,6 g (Ø,279 mol) de l'alcool précédent dans 5ØØ ml de dichlorométhane est refroidie dans un bain d'eau glacée. On y ajoute successivement et sous agitation 2Ø,4 g de 4-diméthylaminopyridine, 63,9 g de p.toluène-sulfochlorure et 38,9 ml de triéthylamine. La solution est maintenue 1 h à température ambiante sous agitation puis diluée par addition d'un litre d'éther. La suspension est filtrée, la phase organique extraite avec une solution à 1Ø% (p/v) de sulfate de cuivre, puis avec une solution saturée en bicarbonate de sodium et enfin avec une solution saturée en chlorure de sodium. Après sèchage et évaporation de l'éther on obtient 47,9 g (71 %) de produit huileux jaune orangé qui est instable et est immédiatement utilisé tel quel pour la suite des synthèses.

**D - Intermédiaires de formule (X)**

**X.1 / benzylisocyanates de formule (II.1)**

Les benzylisocyanates (X) décrits dans les préparations D.1 à D.6 qui suivent sont préparés par réaction de Curtius en une étape à partir d'acides de formule (XIV) précédemment décrits. Instables, ils sont purifiés par chromatographies. Ce sont des huiles visqueuses dont la pureté et l'identité sont vérifiées par les analyses déja décrites. En outre, et plus particulièrement, ces composés présentent en spectrographie infra rouge une bande caractéristique des fonctions isocyanates à 2200-2300cm-1.

Mode opératoire :

Dans un réacteur protégé de l'humidité, à 8 litres de dichlorométhane on ajoute 1,Ø mol d'acide (XIV) à traiter, 162,5 g (2,5 mol) d'azide de sodium et 197,75 g (2Ø2 ml, 2,5 mol) de pyridine. Sous agitation et à température ambiante 263,7 g (187 ml, 1,25 mol) de dichlorophosphate de phényle sont introduits goutte à goutte en 15 minutes.

Le mélange est agité à la température du laboratoire durant 18 à 2Ø h puis extrait successivement à l'eau puis avec HCl 0,1 N. La phase organique est séchée sur sulfate de sodium, celui ci est filtré puis on ajoute au filtrat 5 litres de toluène ; la solution est distillée à la pression ordinaire pour éliminer le dichlorométhane. Le résidu toluènique est chauffé très progressivement et porté au reflux durant 45 minutes à 2 h, jusqu'à fin de dégagement gazeux. Après refrodissement on ajoute de l'hexane, filtre le mélange puis élimine les solvants

par distillation sous vide et sur bain marie. Le résidu coloré, huileux est purifié par chromatographie.

**D.1 / α-cinnamyl-α-éthyl-benzylisocyanate.**

R1 = R5 = C6H5 ; R2 = C2H5)
Rdt = 94% CCM : ∅,45 - ∅,55 ; S.C
RMN : ∅,8∅ (t, 3H) ; 2,∅∅ (q,2H) ; 2,8∅ (d,2H) ; 6,∅∅ (m, 1H) ; (6,5∅ (m,1H) ; 7,25 (m,5H) ; 7,35 (m,5H)

**D.2 / α-cinnamyl-α-méthyl-benzylisocyanate.**

R1 = R5 = C6H5 ; R2 = CH3)
Rdt = 72 % CCM : ∅,5∅ ; S.F
RMN : 1,8∅ (s, 3H) ; 2,6∅-2,85 (m, 2H) ; 5,8∅-6,6∅ (m, 2H) 7,1∅-7,5∅ (m, 1∅H)

**D.3 / α-cinnamyl-α-isopropyl-benzylisocyanate.**

R1 = R5 = C6H5 ; R2 = CH(CH3)2)
Rdt = 95 % CCM : ∅,8∅ ; S.F
RMN : ∅,8∅ (d, 3H) ; 1,2∅ (d,3H) ; 2,∅∅-2,5∅ (m, 1H) ; 3,∅∅ (d, 2H) ; 5,5∅-6,7∅ (m, 2H) ; 7,∅∅ -7,7∅ (m, 1∅H) ;

**D.4 / α-(3′4′-dichloro)cinnamyl-α-éthyl-p.méthoxy-benzylisocyanate**

R1 = p.CH3∅-C6H4 ; R2 = C2H5 ; R5 = 3,4 Cl2-C6H3)
Rdt = 89% CCM : ∅,3∅ ; S.F
RMN : ∅,8∅ (,3H) ; 2,∅∅ (q,2H) ; 2,75 (d,2H) ; 3,8∅ (s,3H) 5,7∅-6,45 (m,2H) ; 6,8∅-7,35 (m,7H)

**D.5 / α-(3′,4′-dichloro) cinnamyl-α-éthyl-p. chloro-benzylisocyanate**

R1 = p.Cl-C6H4 ; R2 = C2H5 ; R5 = 3,4 Cl2-C6H3)
Rdt = 87 % CCM : ∅,5∅ ; S.F
RMN : ∅,8∅ (t,3H) ; 1,95 (q,2H) ; 2,75 (d,2H)) ; 5,7∅ - 6,45 (m,2H) ; 6,9∅-7,6∅ (m,7H)

**D.6 / α-(3′,4′-dichloro) cinnamyl-α-éthyl-m.trifluorométhylbenzylisocyanate**

R1 = m.F3C-C6H4 ; R2 = C2H5 ; R5 = 3,4 Cl2-C6H3)
Rdt = 86 % CCM : ∅,4∅ ; S.F
RMN : ∅,85 (t,3H) ; 2,∅5 (q,2H) ; 2,8∅ (d,2H) ; 5,7∅ - 6,45 (m,2H) ; 6,95 - 7,7∅ (m,7H)

**E - Intermédiaires de formule (V)**

**E.1. : α-cinnamyl-α-éthyl-benzylamine.**

(R1 = R5 = C6H5; R2 = C2H5)

58,∅ g (∅,2∅9 mol) du précurseur α-cinnamyl-α-éthyl-benzylisocyanate décrit en D.1 sont ajoutés à un mélange de 1,5 l de tetrahydrofurane, ∅,2 l d'eau et 5∅ ml d'acide chlorhydrique concentré (d = 1,19). La solution sous agitation est chauffée au reflux durant 18 heures.

Après refroidissement, le THF est éliminé par distillation sous vide et sur bain-maire. Au résidu, on ajoute 2∅∅ ml d'eau, alcalinise le mélange à froid jusqu'à pH 1∅ par ajout de solution concentrée d'hydroxyde de sodium puis extrait à trois reprises par 15∅ ml d'éther. Les phases éthérées sont réunies, lavées à l'eau puis déshydratées sur sulfate de magnésium.

L'éther est éliminé par distillation, le résidu huileux de couleur orangée (54,8 g) est purifié par distillation sous vide. Le produit est obtenu sous forme d'huile incolore visqueuse.
Eb/0,01 = 135-15∅°C. Poids = 38,∅ g Rdt = 72 %
CCM : ∅,15-∅,2∅ ; S.C.
RMN : ∅,7∅ (t,3H); 1,45 (s,2H éch. D2∅); 1,8∅ (m,2H); 2,6∅ (m,2H); 6,∅∅(m,1H); 6,45 (m,1H) ; 7,1∅-7,55 (m,1∅H)

**E.2a) : (+) α-cinnamyl-α-éthyl-benzylamine**

(R1 = R5 = C6H5 ; R2 = C2H5)

Dans un réacteur de 6 litres, on introduit 4,Ø litres d'eau déminéralisée que l'on chauffe au reflux sous agitation. On ajoute alors 213,Ø g (Ø,487 mol) de (±) α-cinnamyl-α-éthyl-benzylamine de l'exemple précédent E.1 puis 139,8 g (Ø,932 mol) d'acide l-(-) tartrique. Le mélange est maintenu au reflux durant 3Ø minutes puis filtré à chaud sur Buchner.

La solution légèrement trouble est abandonnée une nuit pour cristallisation.

Le précipité formé est filtré et séché sous vide à 6Ø°C jusqu'à poids constant. On obtient 127,Ø g de produit. Le filtrat est conservé pour traitements.

Le produit est recristallisé dans 1,3 l d'eau déminéralisée au reflux. Après une nuit de repos, le précipité est filtré puis séché sous vide. On obtient 115,7 g de produit. Le second filtrat est également conservé.

On recristallise de la même façon que précédemment : il est obtenu 92,4 g de produit (Ø,23Ømol). Le troisième filtrat est aussi conservé.

-Pureté optique des produits :

Des prélèvements de l'insoluble purifié et des seconds et troisièmes filtrats de cristallisation sont traités par une solution d'hydroxyde de sodium puis extraits à l'éther. Après évaporation de l'éther, le pouvoir rotatoire spécifique des résidus est déterminé par polarimétrie.

- Résultats.

Filtrat de second cristallisation
$[\alpha]_D^{25}$ = + 12,3° (c = 6,33 ; MeOH)
Filtrat de troisième cristallisation
$[\alpha]_D^{25}$ = + 36,9° (c = 6,4Ø ; MeOH)
Précipité de troisième cristallisation
$[\alpha]_D^{25}$ = + 4Ø,Ø° (C = 6,26 ; MeOH)

Le précipité obtenu après la troisième cristallisation est considéré de pureté optique satisfaisante :
Poids = 92,4 g F = 158°C Rdt = 54,3 %
Anal. C18H21N, C4H6O4) C, H, N, O

Le produit est ajouté à 1 litre d'eau.

Le mélange est alcalinisé sans dépasser 25°C par addition d'hydroxyde de sodium puis extrait à l'éther. Les phases éthérées, lavées et séchées sur Na2SO4 sont évaporées. Le produit intermédiaire(V) dextrogyre est obtenu sous forme d'huile jaune pâle.

Poids = 55,5 g Rdt = 96 %
$[\alpha]_D^{25}$ = + 4Ø,Ø° (c = 6,26 ; MeOH)
RMN (base) : Ø,7Ø (t, 3H) ; 1,45 (s, 2H éch. D2Ø) ; 1,8Ø (m, 2H) ; 2,6Ø (m, 2H) ; 6,ØØ (m, 1H) ; 6,45 (m, 1H) ; 7,1Ø-7,55 (m, 1ØH)

**E.2b :(-)α-cinnamyl-α-éthyl-benzylamine.**

(R1 = R5 = C6H5 ; R2 = C2H5 ; R3 = R4 = H)

Le filtrat obtenu à la première cristallisation du produit E.2a) précédent est alcalinisé par une solution concentrée d'hydroxyde de sodium puis extrait par 3 fois 5ØØ ml d'éther. les phases éthérées réunies sont lavées par une solution saturée en chlorure de sodium puis déshydratées sur Na2SO4. L'éther est éliminé par distillation.

Poids de résidu : 135,Ø g
$[\alpha]_D^{25}$ = - 2Ø,6° (c = 5,7 ; MeOH)

Le produit est introduit au reflux dans 2,3 l d'eau et on ajoute 88,7 g (Ø,59 mol) d'acide d-(+) tartrique. Après dissolution et filtration pour éliminer les impuretés mécaniques, la solution est abandonnée une nuit.

Le précipité formé est filtré et séché à 6Ø°C sous vide. Poids : 149,Ø g. Le filtrat de première cristallisation est conservé.

L'insoluble est recristallisé dans 1,5 l d'eau au reflux ; après une nuit de repos, le précipité est filtré et séché. Poids : 127,Ø g. Le filtrat est conservé.

- Pureté optique des produits.

On procède de la même façon que décrit précédemment avec un échantillon des filtrats de première et de seconde cristallisation ainsi qu'avec le produit obtenu après la seconde cristallisation.

- Résultats.

Filtrat de première cristallisation
$[\alpha]_D^{25}$ = + 18,0° (c = 6,30 ; MeOH)
Filtrat de seconde cristallisation

$[\alpha]_D^{25}$ = - 23,0° (c = 6,10 ; MeOH)
Précipité de seconde cristallisation
$[\alpha]_D^{25}$ = - 39,3° (c = 5,5 ; MeOH)

Ce dernier produit est considéré de pureté optique satisfaisante et comparable à celle de l'isomère dextrogyre obtenu précédemment.
Poids : 127,0 g F = 158°C Rdt = 74,7 %
Anal. ($C_{18}H_{21}N$, $C_4H_6O_4$) C, H, N, O

Le tartrate est ajouté à 1,5 litre d'eau et à une température inférieure à 25°C, alcalinisé par une solution concentrée d'hydroxyde de sodium puis extrait à l'éther. Les phases éthérées réunies sont traitées et après évaporation, le produit (V) lévogyre est obtenu sous forme d'huile jaune pale.
Poids = 75,0 g Rdt = 94 %
$[\alpha]_D^{25}$ (base) = - 39,3° (c = 5,5 ; MeOH)
RMN (base) : 0,70 (t, 3H) ; 1,45 (s, 2H éch. $D_2O$) ; 1,80 (m, 2H) ; 2,60 (m, 2H) ; 6,00 (m, 1H) ; 6,45 (m, 1H) ; 7,10-7,55 (m, 10H)

**F - INTERMEDIAIRES DE FORMULE (VII)**

**F.1 / (-)α-cinnamyl-α-éthyl-N-méthyl benzylamine.**

($R_1$ = $R_5$ = $C_6H_5$ ; $R_2$ = $C_2H_5$ ; $R_3$ = $CH_3$)

Dans un réacteur protégé de l'humidité et sous atmosphère d'azote, on introduit 182 ml de THF anhydre, 7,7 g (0,167 mol) d'acide formique pur et 26,6 g (0,164 mol) de 1,1'-carbonyldiimidazole.

La solution est agitée une heure à température ambiante puis on ajoute 40,0 g (0,159 mol) de (+) α-cinnamyl-α-éthyl-benzylamine obtenue en E.2a en solution dans 430 ml de THF anhydre.

L'introduction est réalisée en 15 minutes à température ambiante puis l'agitation est maintenue durant 4 heures.

Le THF est éliminé par distillation sous vide et sur bain-marie et le résidu est repris par 300 ml de solution HCl N. Le mélange est extrait à l'éther et la phase acide écartée.

La phase éthérée est extraite par une solution saturée en bicarbonate de sodium, puis à l'eau et enfin déshydratée sur $Na_2SO_4$. L'éther est concentré jusqu'à un volume résiduel de 200 ml. Cette solution résiduelle éthérée est maintenue 24 h à 4°C.

L'insoluble cristallin qui précipite est le (+) α-cinnamyl-α-éthyl-N-formyl-benzylamine (intermédiaire XI; $R_1$ = $R_5$ = $C_6H_5$ ; $R_2$ = $C_2H_5$ ; $R_7$ = H)

Il est filtré, séché sous vide à 60°C jusqu'à poids constant.
Poids : 41,0 g F = 101°C Rdt = 92,3%
$[\alpha]_D^{25}$ = + 38,5° (c = 4,05 ; MeOH)

Dans un réacteur protégé de l'humidité et sous atmosphère d'azote, on introduit 25,5 g (0,192 mol) de chlorure d'aluminium. Le réacteur est refroidi par un bain de carboglace/acétone et on ajoute 309 ml d'éther anhydre en 15 minutes environ et sans dépasser 0°C. On obtient une solution qui est maintenue 30 minutes à 0°C.

Parallèlement, dans un réacteur également protégé de l'humidité et sous atmosphère d'azote, il est introduit 22,3 g (0,587 mol) d'hydrure de lithium aluminium et après refroidissement par un bain carboglace/acétone, 309 ml de THF anhydre en 10 minutes environ.

La suspension obtenue est maintenue sous agitation durant 15 minutes à 0°C puis transférée dans le premier réacteur contenant la solution éthérée de chlorure d'aluminium.

Le transfert est effectuée en 10 minutes à une température inférieure à 0°C.

Après 3Ø minutes d'agitation, on y ajoute 41,Ø g (Ø,147 mol) de l'intermédiaire précédent en solution dans 12Ø ml de THF anhydre, en 3Ø minutes et à une température voisine de Ø°C.

Après retour vers 2Ø°C, le mélange réactionnel est porté au reflux, sous agitation durant 2 heures.

On refroidit à Ø°C puis introduit goutte à goutte 47,8 ml d'une solution d'hydroxyde de sodium à 1Ø % (p/v) puis 42 ml d'eau.

Le mélange est abandonné une nuit, l'insoluble filtré sur Buchner et lavé au THF. Les filtrats réunis sont évaporés sous vide et sur bain-marie. On obtient un résidu brut huileux.

Poids : 36,1 g

Le produit est purifié par chromatographie sur colonne de silice. L'élution par un mélange chlorure de méthylène-méthanol 98-2 (v/v) permet d'obtenir le produit purifié sous forme d'une huile incolore.

Poids : 32,6 g    Rdt = 83,5 %    CCM : Ø,45-Ø,55 ; S.L.

[α] $^{25}_{D}$ = - 16,4° (c = 6,1 ; MeOH)

RMN (base) : Ø,7Ø (t, 3H) ; 1,4Ø (s, 1H éch. D2Ø) ; 1,8Ø (q,2H) ; 2,2Ø (s, 3H) ; 2,7Ø (d, 2H) ; 5,75-6,5Ø (m, 2H); 7,1Ø-7,6Ø (m, 1ØH)

**F.2 / <u>(+) α-cinnamyl-α-éthyl-N-méthyl benzylamine.</u>**

(R1 = R5 = C6H5 ; R2 = C2H5 ; R3 - CH3)

En procédant de façon identique à celle décrite en F.1 et à partir de 4Ø,Ø g de (-) α-cinnamyl-α-éthyl-benzylamine préparée en E.2b, on obtient :

**<u>(-)α-cinnamyl-α-éthyl-N-formyl benzylamine.</u>**

(Intermédiaire XI - R1 = R5 = C6H5 ; R2 = C2H5 ; R7 = H)

Poids : 39,3 g    F = 1Ø1°C    Rdt = 88,5 %

[α] $^{25}_{D}$ = - 37,9° (c = 5,82 ; MeOH)

Puis après réduction de 39,Ø g (Ø,14Ø mol) de ce composé et tel que décrit en F.1, on obtient à l'état brut 36,4 g de produit qui sont purifiés par chromatographie sur colonne de silice.

Poids : 31,3 g    Rdt = 84,2 %    CCM : Ø,45-Ø,55 ; S.L.

[α] $^{25}_{D}$ = + 16,3° (c = 5,58 ; MeOH)

RMN : Ø,7Ø (t, 3H) ; 1,4Ø (s, 1H éch. D2Ø) ; 1,8Ø (q, 2H); 2,2Ø (s, 3H) ; 2,7Ø (d, 2H) ; 5,75-6,5Ø (m, 2H) ; 7,1Ø-7,6Ø (m, 1ØH)

**F.3 : <u>(+/-) α-cinnamyl-α-éthyl-N-méthyl-benzylamine.</u>**

(R1 = R5 = C6H5 ; R2 = C2H5 ; R3 =CH3)

Dans un réacteur de 12 l refroidi dans un bain de glace, on introduit sous atmosphère d'azote, 145,Ø g (1,Ø9 mol) de chlorure d'aluminium.

Lentement et avec précautions, on ajoute 9ØØ ml de THF anhydre.

A la solution rouge foncé obtenue, on ajoute 3,2 l de solution 1 M d'hydrure de lithium aluminium (3,2 mol) dans le THF. Le mélange est agité 15 minutes puis on ajoute goutte à goutte et sans dépasser 5°C, 233,Ø g (Ø,84 mol) d'-α-cinnamyl-α-éthyl-benzylisocyanate en solution dans 2ØØ ml de THF.

Après introduction le mélange est maintenu sous agitation 4 h à la température du laboratoire puis refroidi à 5°C environ par un bain de glace.

On ajoute 1,8 l d'éther méthyl-t.butylique puis goutte à goutte, avec précautions, 195 ml de solution de NaOH à 1Ø % (p/v). On ajoute ensuite 24Ø ml d'eau et laisse agiter le mélange 16 h à la température ambiante.

La suspension est filtrée sur buchner et sous vide. L'insoluble est écarté et le filtrat concentré par distillation sous vide et sur bain marie. Le produit brut résiduel est une huile visqueuse jaune qui est purifiée par distillation. Produit purifié : Eb/Ø,Ø2 = 135-15Ø°C, qui cristallise dans l'hexane.

Poids = 183,2 g    F = 54-56°C    Rdt = 82 %

CCM : Ø,45-Ø,55; S.C.

RMN : Ø,7Ø (t, 3H); 1,4Ø (s, 1H ech. D2Ø); 1,8Ø (q,2H); 2,2Ø (s,3H); 2,7Ø (d,2H); 6,ØØ-6,45 (m,2H); 7,1Ø-7,6Ø (m, 1ØH)

Selon le mode opératoire de cette préparation F.3 et à partir des benzylisocyanate substitués de façon appropriée, les N-méthyl benzylamines F.4 à F.8 sont préparées :

**F.4 : (+/-) α-cinnamyl-α-méthyl-N-méthyl-benzylamine.**

(R1 = R5 = C6H5 ; R2 = R3 =CH3)
A partir d'α-cinnamyl-α-méthyl-benzylisocyanate
Rdt = 78 % (chromatographie) CCM : Ø,4Ø-Ø,5Ø ; S.M. RMN : 1,45 (s, 3H); 1,55 (s, 1H ech. D2Ø); 2,2Ø (s,3H); 2,6Ø (d,2H); 5,75-6,5Ø (m,2H); 7,15-7,45 (m, 1ØH)

**F.5 : (+/-)α-cinnamyl-α-isopropyl-N-méthyl-benzylamine.**

(R1 = R5 = C6H5 ; R2 = CH(CH3)2 ; R3 = CH3)
A partir d'α-cinnamyl-α-cinnamyl-α-isopropyl-benzylisocyanate
Rdt = 65 % F = 62°C (éth. petr.) CCM : Ø,7Ø : S.M.
RMN : Ø,75 (d, 3H); Ø,85 (d, 3H) ; 1,45 (s, 1H ech, D2Ø); 1,7Ø-2,1Ø (m,1H); 2,3Ø (s,3H); 2,85-3,Ø5 (m,2H); 6,Ø5-6,7Ø (m,2H); 7,2Ø-7,6Ø (m, 1ØH)

**F.6 : (+/-) α-(3',4'-dichloro)cinnamyl-α-éthyl-N-méthyl-p. méthoxybenzylamine.**

(R1 = p. CH3Ø-C6H4 ; R2 = C2H5 ; R3 = CH3 ; R3 =CH3 ; R5 = 3,4 C12-C6H3)
A partir d'α-(3',4'-dichloro)cinnamyl-α-éthyl-p. méthoxy- benzylisocyanate
Rdt = 4Ø % (chromatographie) CCM : Ø,55 ; S.L.
RMN : Ø,7Ø (t, 3H); 1,4Ø (s, 1H ech. D2Ø); 1,6Ø-1,9Ø (q,2H); 2,15 (s,3H); 2,6Ø (d,2H); 3,8Ø (s, 3H) ; 5,7Ø-6,4Ø (m,2H); 6,7Ø-7,4Ø (m, 7H)

**F.7 : (+/-) α-(3', 4'-dichloro)cinnamyl-α-éthyl-N-méthyl-p. chlorobenzylamine.**

(R1 = p. Cl-C6H4 ; R2 = C2H5 ; R3 = CH3 ; R5 = 3,4 Cl2-C6H3)
A partir d'α-(3',4'-dichloro)cinnamyl-α-éthyl-p. chloro-benzylisocyanate
Rdt = 86 % (brut) CCM : Ø,5Ø ; S.L.
RMN : Ø,7Ø (t, 3H); 1,35 (s, 1H ech. D2Ø); 1,8Ø (q,2H); 2,2Ø (s,3H); 2,65 (d,2H); 5,7Ø-6,5Ø (m,2H); 6,9Ø-7,5Ø (m, 7H)

**F.8 : (+/-) α-(3',4'-dichloro)cinnamyl-α-éthyl-N-méthyl-m. trifluorométhylbenzylamine.**

(R1 = m.F3C-C6H4 ; R2 = C2H5 ; R3 = CH3 ; R5 = 3,4 Cl2-C6H3)
A partir d' α-(3',4'-dichloro)cinnamyl-α-éthyl-m. trifluorométhylbenzylisocyanate.
Rdt = 88 % (brut) CCM : Ø,7Ø ; S.L.
RMN : Ø,75 (t, 3H); 1,4Ø (s, 1H ech. D2Ø); 1,85 (q,2H); 2,2Ø (s,3H); 2,7Ø (d,2H); 5,7Ø-6,45 (m,2H); 6,95-7,85 (m, 7H)

**G - INTERMEDIAIRE DE FORMULE (IX)**

**G.1 : α-amino-N-cyclopropylméthyl-N-méthyl-α-(3',4',5'-triméthoxy)cinnamyl-phénylacétonitrile**

(R1 = C6H5 ; R3 =CH3 ; m = 1; R4 = (CH2)2CH ; R5 = 3,4,5 (CH3Ø)3-C6H2)

Dans un réacteur protégé de l'humidité et sous atmosphère d'azote, 1,Ø25 mole de n. butyllithium (solution 1ØM/ hexanes) est ajoutée goutte à goutte à 2Ø°C dans une solution de 1,Ø25 mole de diisopropylamine dans 1 litre de tetrahydrofurane anhydre.

Le mélange est maintenu 15 minutes à 2Ø°C. A - 72°C, il est introduit 2ØØ,3 g (1,Ø mole) d'α-amino-N-cyclopropylméthyl-N-méthyl-phénylacétonitrile en solution dans 2ØØ ml de THF, l'agitation est maintenue 1 h 3Ø à cette température puis on ajoute 113,Ø g (1,Ø25 mole) de chlorure de 3',4',5'-triméthoxy-cinnamyle en solution dans 5ØØ ml de THF. Après 2Ø minutes à - 72°C le mélange est agité 1 h à température ambiante.

On ajoute ensuite 1,5 l de solution de NH4Cl à 1Ø % (p/v) et 75Ø ml de mélange hexanes-acétate d'éthyle 1-1 (v/v).

La phase organique est séparée, la phase aqueuse réextraite par le mélange de solvants. les phases organiques réunies sont lavées par extraction avec une solution saturée en chlorure de sodium, puis séchées sur MgSO4. Les solvants sont éliminés par distillation sous vide et sur bain-maire. le produit brut huileux (33Ø,Ø g) est purifié par cristallisation.
Poids : 222,7 g F = 67°C (hexanes) Rdt = 55 %

RMN : 2,1Ø-2,35 (m, 4H); 2,45-2,6Ø (m, 5H) ; 2,65-3,Ø5 (m, 1H) ; 3,7Ø (s, 2H) ; 3,8Ø (s, 9H) ; 5,15-5,65 (m, 2H) 6,1Ø-6,4Ø (m,2H); 7,2Ø-7,65 (m, 5H)

**PRODUITS DE L'INVENTION : EXEMPLES**

**Exemple 1 : α-cinnamyl-N-cyclopropylméthyl-α-éthyl benzylamine**

(II ; R1 = R5 = C6H5 ; R2 = C2H5 ; m = 1 ; R4 = CH (CH2)2)

a) Dans un réacteur protégé de l'humidité on introduit successivement 16Ø ml de chlorure de méthylène, 1Ø,Ø g (4Ø mmol) d'α-cinnamyl-α-éthyl-benzylamine (intermédiaire V décrit à la préparation E.1) et 5,6 ml (4 g, 4Ø mmol) de triéthylamine.

Sous atmosphère d'azote et à une température inférieure à 2Ø°C, on introduit en 2Ø minutes 3,6 ml (4,16 g, 4Ø mmol) du chlorure de l'acide cyclopropane carboxylique.

Le mélange est maintenu sous agitation 2 h 3Ø à 2Ø°C puis extrait successivement par une solution à 1Ø % d'ammoniaque, puis à l'eau, puis par une solution saturée en bicarbonate de sodium et enfin à nouveau à l'eau puis séchée sur Na2SO4.

Le solvant est évaporé et l'intermédiaire N-cyclopropyl carboxamide correspondant (IV) est purifié par cristallisation dans l'éther.

| | |
|---|---|
| Poids : 10,2 g | Rdt = 80 % |
| F = 130°C | CCM : 0,80 ; S.H. |

RMN : Ø,4Ø-1,ØØ (m, 7H) ; 1,ØØ-1,5Ø (m, 1H) ; 1,9Ø-2,3Ø (m, 2H) ; 2,8Ø-3,2Ø (m, 2H) ; 5,8Ø-6,5Ø (m, 3H dont 1H ech. D2Ø) ; 7,ØØ-8,ØØ (m, 1ØH).

b) Dans un réacteur "a" protégé de l'humidité on introduit 4,42 g (33 mmol) de chlorure d'aluminium anhydre puis, en refroidissant par un bain de carboglace/acétone, on ajoute, en 5 minutes environ 56 ml d'éther déshydraté sur tamis moléculaire. La solution est agitée 3Ø minutes à Ø°C.

D'autre part, dans un réacteur "b" également protégé de l'humidité et sous atmosphère d'azote, on introduit 3,8 g (1ØØ mmol) de LAH puis après refroidissement par un bain carboglace/acétone, on ajoute en 1Ø minutes environ et sans dépasser Ø°C, 5Ø ml de THF déshydraté sur tamis. Le mélange est agité 15 minutes à Ø°C.

La suspension préparée en "b" est introduite à Ø°C et en 3Ø minutes environ dans la solution éthérée de chlorure d'aluminium du réacteur "a".

A la température ambiante, on ajoute alors 1Ø,1 g (32 mmol) du N-cyclopropylcarboxamide intermédiaire en solution dans 56 ml de THF. Le mélange est porté 1 heure au reflux et après refroidissement à 2Ø°C on ajoute avec précautions 6,Ø ml de solution NaOH à 1Ø % puis 7,5 ml d'eau.

Après une nuit d'agitation l'insoluble est filtré et le filtrat évaporé sous vide. Le produit brut est obtenu sous forme visqueuse (8,8 g). Il est purifié par chromatographie.

Poids : 5,Ø g    Rdt = 51 %    CCM : Ø,45 ; S.L.

RMN : Ø,ØØ-Ø,1Ø (m, 2H) ; Ø,3Ø-Ø,6Ø (m,2H) ; Ø,6Ø-1,ØØ (m, 4H) ; 1,6Ø-2,ØØ (m,3H) ; 2,2Ø (d, 2H) ; 2,65 (d, 2H) ; 5,8Ø-6,6Ø (m, 2H) ; 7,ØØ-7,7Ø (m, 1ØH) dont 1H ech. D2Ø)

Chlorhydrate :

Rdt = 82 %    F = 2ØØ°C (éther)
Anal (C22H27N, HCl) C, H, Cl, N

**Exemple 2 : α-cinnamyl-α-éthyl-N-(1-méthylcyclopropyl)méthyl-benzylamine**

(II ; R1 = R5 = C6H5 ; R2 = C2H5 ; m = 1 ; R4 = (C(CH2)2CH3)

Dans un réacteur protégé de l'humidité, on dissout dans 4Ø,Ø ml de diméthylformamide déshydraté sur tamis moléculaire, 5,Ø g (2Ø mmol) d'α-cinnamyl-α-éthyl-benzylamine (préparation E.1).

A la solution, on ajoute successivement sous atmosphère d'azote :

- 2,1 g (21 mmol) d'acide 1-méthyl cyclopropanecarboxylique,
- 2,8 g (21 mmol) d'hydroxybenzotriazole,
- 4,3 g (2Ø mmol) de dicyclohexylcarbodiimide, et 11,2 ml de triéthylamine anhydre.

La solution est maintenue sous agitation 24 heures à la température du laboratoire puis évaporée sous

vide et sur bain marie.

Le résidu est dissout dans 15∅ ml d'acétate d'éthyle. La solution extraite par 15∅ ml d'une solution à 1∅ % (p/v) d'acide citrique et le précipité formé filtré.

La phase aqueuse est écartée. La phase organique est traitée, séchée sur Na2SO4 et l'acétate d'éthyle est éliminé par distillation. Le résidu est cristallisé dans l'hexane, l'insoluble qui est le N-carboxamide (IV) correspondant est filtré et séché.

Poids : 4,7 g Rdt = 7∅ % F = 1∅∅°C CCM : ∅,45 ; S.L.

RMN : ∅,4∅-∅,6∅ (m, 2H) ; ∅.75 (t, 3H) ; 1,∅∅-1.3∅ (m, 2H) ; 1,3∅ (s, 3H) ; 1,9∅-2,3∅ (m, 2H) ; 2,7∅-3,2∅ (m, 2H) ; 5,6∅-6,6∅ (m, 3H dont 1H ech. D2∅) ; 6,9∅-7,5∅ (m, 1∅ H)

b) 4,5 g (13,5 mmol) du N-carboxamide précédent sont réduits tel que décrit en b) de l'exemple 1.

Rdt = 93 % (chromatographie) CCM : ∅,8∅ ; S.L.

RMN : ∅,2∅ (s, 4H) ; ∅,7∅ (t, 3H) ; 1,1∅ (s, 3H) ; 1,3∅ (s, 1H ech. D2∅) ; 1,5∅-1,9∅ (m, 2H) ; 2,∅∅-2,3∅ (m, 2H) 2,65 (d, 2H) ; 5,7∅-6,5∅ (m, 2H) ; 6,9∅-7,6∅ (m, 1∅ H)

Chlorhydrate :

Rdt = 85 % F = 212-214°C (éther)

Anal. (C23H3∅Cl N.HCl) C, H, Cl, N

**Exemple 3 : α-cinnamyl-α-éthyl-N-(trans 2-phénylcyclopropyl)méthyl-benzylamine**

(II, R1 = R5 = C6H5 ; R2 = C2H5 ; m = 1 ; R4 = C6H5-CH (CH2)CH). Le composé est préparé selon le mode opératoire de l'exemple 1 à partir de l'intermédiaire V décrit à la préparation E.1 et du chlorure de l'acide trans 2-phényl-1 cyclopropanecarboxylique

a) Intermédiaire IV (m = 1 ; R4 = C6H5-CH(CH2)CH)

Rdt = 95 % F = 163°C (Hexanes-chlor. méthylène)

CCM : ∅,3∅-∅,4∅ ; S.G.

b) Composé II de l'exemple

Rdt = 83 % (brut) CCM : ∅,5∅-∅,6∅ ; S.K.

RMN : ∅,6∅-1,∅∅ (m, 5H) ; 1,∅∅-1,95 (m, 5H dont 1H éch.D2∅) ; 2,2∅-2,5∅ (m, 2H) ; 2,7∅ (d, 2H) ; 5,8∅-6,6∅ (m, 2H) ; 6,9∅-7,55 (m, 15 H)

Chlorhydrate :

Rdt = 72 % F = 217°C (éther)

Anal. (C28H31N,HCl) C, H, Cl, N

**Exemple 4 : α-cinnamyl-N-cyclohexylméthyl-α-éthyl-benzylamine**

(II ; R1 = R5 = C6H5 ; R2 = C2H5 ; m = 1 ; R4 = CH (CH2)5)

a) Dans un réacteur protégé de l'humidité on introduit 34∅ ml de THF déshydraté sur tamis moléculaire et 7,7 g (6∅ mmol) d'acide cyclohexanecarboxylique. On ajoute ensuite à cette solution 7,3 ml (6,7 g - 66 mmol) de N-méthyl morpholine.

Après 5 minutes d'agitation, la solution est refroide à -2∅°C puis on y ajoute en 3∅ minutes environ et à -2∅°C, 7,8 ml (8,2 g - 6∅ mmol) de chloroformiate d'isobutyle et 15,∅ g (6∅ mmol) de la benzylamine préparée en E.1.

La suspension est agitée 3∅ minutes à -2∅°C puis 16 heures à la température ambiante. Après concentration par distillation sous vide jusqu'à obtention d'un concentrat de 25∅ ml environ on ajoute 3∅∅ ml d'éther et extrait successivement par une solution HCl 2N, une solution saturée en NaHCO3 puis une solution saturée en NaCl.

La phase organique est séchée, évaporée sous vide.

Le résidu de produit brut (12,4 g) est purifié par chromatographie pour obtenir l'intermédiaire (IV) correspondant (m = 1, R4 = CH(CH2)5)

Poids = 1∅,3 g Rdt = 48 % CCM : ∅,3∅ ; S.G.

RMN : ∅,7∅ (t, 3H) ; 1,∅∅-2,3∅ (m, 13 H) ; 2,7∅-3,2∅ (m, 2H) ; 5,5∅-6,6∅ (m, 3H dont 1H éch.D2∅) ; 7,∅∅-7,6∅ (m, 1∅H).

b) Le N-cyclohexylcarboxamide précédent est réduit comme décrit au stade b) de l'exemple 1 :

Rdt = 7∅ % (chromatographie) CCM : ∅,7∅ ; S.H.

RMN : Ø,7Ø (t, 3H) ; 1,ØØ-2,ØØ (m, 14 H dont 1H ech. D2Ø) ; 2,2Ø (d, 2H) ; 2,7Ø (d, 2H) ; 5,8Ø-6,5Ø (m, 2H) ; 7,ØØ-7,6Ø (m, 1ØH).

**Exemple 5 : α-cinnamyl-N-cyclopropyléthyl-α-éthyl benzylamine**

(II ; R1 = R5 = C6H5 ; R2 = C2H5 ; m = 2 ; R4 = CH (CH2)2)

a) l'intermédiaire N-carboxamide correspondant (IV ; m = (2) ; R4 = CH (CH2)2) est préparé selon le stade a) de l'exemple 4 précédent à partir d'α-cinnamyl-α-éthylbenzylamine (préparation E.1) et d'acide 2-cyclopropane acétique.

Rdt = 75 % (brut) CCM : Ø,8Ø ; S.H.

RMN : Ø,ØØ-Ø,3Ø (m, 2H) ; Ø,4Ø-1,ØØ (m, 6H) ; 2,ØØ-2,4Ø (m, 4 H) ; 2,8Ø-3,1Ø (m, 2H) ; 5,7Ø-6,6Ø (m, 3H dont 1H ech. D2Ø) ; 7,1Ø-7,6Ø (m, 1ØH)

b) Le N-carboxamide précédent est réduit selon le mode opératoire b) de l'exemple 1

Rdt = 83 % CCM : Ø,7Ø ; S.H.

RMN : Ø,ØØ-Ø,1Ø (m, 2H) ; Ø,2Ø-Ø,5Ø (m, 2H) ; Ø,5Ø-Ø,9Ø (m, 4 H) ; 1,1Ø-1,5Ø (m, 2H) ; 1,5Ø-2,ØØ (m, 3H dont 1H ech. D2Ø) ; 2,3Ø-2,8Ø (m, 4H) ; 5,8Ø-6,6Ø (m, 2H) ; 7,1Ø-7,6Ø (m, 1ØH)

**Exemple 6 : α-cinnamyl-α-éthyl-N-méthyl-N-(trans 2-phénylcyclopropyl)méthyl-benzylamine**

(III ; R1 = R5 = C6H5 ; R2 = C2H5 ; R3 = CH3 ; m = 1 ; R4 = C6H5-CH (CH2)CH)

Dans un réacteur, on introduit 15Ø ml d'acétonitrile et 8,5 g (22 mmol) du composé II obtenu à l'exemple 3. on ajoute ensuite 28,Ø ml de solution de formaldéhyde à 37 % (p/v) (1Ø,36 g - 518 mmol) puis refroidit le mélange à Ø°C.

A cette température, on ajoute alors 4,2 g (67 mmol) de cyanoborohydrure de sodium et laisse agiter 5 minutes, et ajoute goutte à goutte en 1Ø minutes environ, 4,2 ml (4,41 g - 73 mmol) d'acide acétique pur.

Le mélange est agité une heure à température ambiante puis on ajoute successivement à la même température, 1,4 g (24 mmol) d'acide acétique. On laisse agir une heure puis renouvelle l'addition de ces réactifs en quantités identiques.

Après une dernière période d'agitation de 3 heures à la température ambiante, on ajoute 3ØØ ml d'éther, élimine la phase aqueuse et lave la phase organique par extractions successives avec 1ØØ ml de solution NaOH 1Ø %, 2 fois 1ØØ ml d'eau puis 2 fois 2ØØ ml de solution saturée en ClNa. La phase organique est séchée sur Na2SO4 et les solvants éliminés par distillation sous vide.

Le produit brut obtenu (8,Ø g) est purifié par chromatographie (6,Ø8 g) et finalement par distillation sous vide : Eb/ Ø,Ø8 = 225-23Ø°C

Poids = 4,5 g Rdt = 52 % CCM : Ø,8Ø ; S.K.

RMN : Ø,5Ø-Ø,9Ø (m, 5H) ; 1,ØØ-1,7Ø (m, 2H) ; 1,9Ø (q,2H) ; 2,2Ø-2,6Ø (m, 5H) ; 2,8Ø (d, 2H) ; 6,ØØ-6,6Ø (m, 2H) ; 6,9Ø-7,55 (m, 15H)

**Exemple 7 : α-cinnamyl-N-cyclohexylméthyl-α-éthyl N-méthyl-benzylamine**

(II ; R1 = R5 = C6H5 ; R2 = C2H5 ; R3 = CH3 ; m = 1 ; R4 = CH (CH2)5)

Le composé est préparé à partir du produit de l'exemple 4 par méthylation réductrice avec le formaldéhyde et le cyanoborohydrure de sodium tel que décrit à l'exemple 7 précédent.

Rdt = 8Ø % (chromatographie) CCM : Ø,6Ø ; S.H.

RMN : Ø,7Ø (t, 3H) ; 1,ØØ-2,ØØ (m, 13H) ; 2,2Ø-2,5Ø (m, 2H) ; 2,7Ø-3,ØØ (m, 2H) ; 6,ØØ-6,6Ø (m, 2H) ; 7,1Ø-7,6Ø (m, 1ØH)

**Exemple 8 : α-cinnamyl-N-cyclopropyléthyl-α-éthyl-N-méthyl-benzylamine**

(II ; R1 = R5 = C6H5 ; R2 = C2H5 ; R3 = CH3 ; m = 2 ; R4 = CH (CH2)2)

Le produit est préparé à partir du composé de l'exemple 5 selon le mode opératoire décrit à l'exemple 6.

Rdt = 76 % (chromatographie) CCM : Ø,7Ø ; S.H.

RMN : Ø,ØØ-Ø,1Ø (m, 2H) ; Ø,2Ø-Ø,5Ø (m, 2H) ; Ø,5Ø-Ø,9Ø (m, 4H) ; 1,3Ø (q, 2H) ; 1,7Ø-2,ØØ (m, 2H) ; 2,25 (s, 3H) ; 2,4Ø-2,7Ø (m, 2H) ; 2,8Ø (d, 2H) ; 6,ØØ-6,6Ø (m, 2H) ; 7,1Ø-7,6Ø (m, 1ØH)

**Exemple 9 : α-cinnamyl-N-cyclopropylméthyl-α-éthyl-N-méthyl-benzylamine.**

(III.3; R1 = R5 = C6H5; R2 = C2H5; R3 = CH3 ; m = 1 ; R4 = (CH2)2CH)

Dans un réacteur protégé de l'humidité et sous atmosphère d'azote on dissout dans 2ØØ ml de dichlorométhane 13,52 g (Ø,Ø51 mol) d'α-cinnamyl-α-éthyl-N-méthyl-benzylamine (décrit à la préparation F.3) et 8,5 ml (61 mmol) de triéthylamine. Sous agitation et à température ambiante on ajoute 5,5 ml (Ø,Ø61 mol) de chlorure de l'acide cyclopropane carboxylique.

Le mélange est agité 24 h à température ambiante puis extrait successivement par une solution diluée d'ammoniaque, une solution d'acide chlorhydrique, puis lavé à l'eau et séché sur MgSO4. Après évaporation le dérivé de formule IV (R3 = CH3, m = 1, R4 = CH (CH2)2), est obtenu sous forme d'huile visqueuse de couleur orange et, sans être purifié est réduit dans le stade suivant.

Dans un réacteur protégé de l'humidité et sous atmosphère d'azote, on prépare une solution d'hydrure d'aluminium dans le mélange THF/éther à partir de 6,8Ø g (Ø,Ø51 mol) de chlorure d'aluminium et de 5,8Ø (Ø,Ø15 mol) d'hydrure de lithium aluminium.

Au mélange obtenu, on ajoute l'amide obtenu précédemment en solution dans 1ØØ ml de THF. Le mélange réactionnel est agité 3 h à la température du laboratoire puis dilué par ajout de 25Ø ml d'éther. On ajoute goutte à goutte avec précautions, 9,2 ml de solution NaOH 1Ø % (p/v) puis 11,Ø ml d'eau et laisse agiter 16 h. Le mélange est filtré, le filtrat évaporé sous vide et sur bain marie et le résidu purifié par distillation.

Poids : 13,52 g    Eb/Ø,Ø2 = 15Ø-19Ø°C
Rdt = 83 %
CCM : Ø,7Ø-Ø,75 ; S.E.

RMN : Ø,1Ø (m, 2H); Ø,3Ø-Ø,6Ø (m, 2H); Ø,6Ø-1,ØØ (m, 4H); 1,9Ø (q,2H); 2,35 (d,2H); 2,45 (s,3H); 2,8Ø (d,2H); 6,ØØ-6,5Ø (m,2H) ; 7,1Ø-7,6Ø (m,1ØH)

Hémimaléate :

A une solution éthérée de 4,88 g (15,3 mmol) de produit précédent, on ajoute 1,86 g (16 mmol) d'acide maléique en solution éthanolique. Le précipité qui se forme à froid sous agitation est filtré et séché.

Poids : 5,36 g    Rdt = 8Ø %    F = 99-1Ø2°C
Anal. (C23H29N.C4H4Ø4) C, H, N, Ø

**Exemple 1Ø : Chlorohydrate(+)d'α-cinnamyl-N-cyclopropylméthyl- α -éthyl- N-méthyl-benzylamine.**

(III ; R1 = R5 = C6H5; R2 = C2H5; R3 = CH3; m = 1 ; R4 = (CH2)2CH)

Selon le mode opératoire de l'exemple 1Ø, à partir de 15,4 g (Ø,Ø58 mol) de (-) α-cinnamyl-α-éthyl-N-méthyl-benzylamine (préparation F.1) et de 7,28 g (Ø,Ø7Ø mol) de chlorure d'acide cyclopropane carboxylique, il est obtenu 18,9 g (Rdt = 97,7 %) de l'intermédiaire N-méthyl N-cyclopropylcarboxamide.

[α] $^{25}_{D}$ = + 92,6° (C = 5,94 ; MeOH)

Le produit réduit selon la technique de l'exemple 1Ø permet d'obtenir le produit qui est purifié par chromatographie sur colonne.

Poids : 14,7 g    Rdt : 81 %    CCM : Ø,7Ø-Ø,8Ø ; S.E.
Anal. (C23H19N) C, H, N
RMN : Ø,1Ø (m,2H); Ø,3Ø-Ø,6Ø (m, 2H); Ø,6Ø-1,ØØ (m, 4H); 1,9Ø (q,2H); 2,35 (d,2H); 2,45 (s,3H); 2,8Ø (d,2H); 6,ØØ-6,5Ø (m,2H); 7,1Ø-7,6Ø (m,1ØH)

Chlorhydrate :

Rdt = 83 %    F = 177°C (acet. éthyle)
[α] $^{25}_{D}$ = + 49,7° (C = 3 ; méthanol)
Anal. C23H29N, HCl) C, H, Cl, N

**Exemple 11 : Chlorhydrate (-) d'α-cinnamyl-N-cyclopropylméthyl-α-éthyl-N-méthyl-benzylamine**

(III ; R1 = R5 = C6H5; R2 = C2H5; R3 = CH3; m = 1 ; R4 = (CH2)2CH)

Préparé selon l'exemple 11 précédent, à partir de l'intermédiaire préparé en F.2, on obtient le N-méthyl-N-cyclopropyl-carboxamide correspondant :

Rdt = 94,3 %
[α] $^{25}_{D}$ = - 91,4° (C = 5,6 % MeOH)

qui, réduit selon la technique de l'exemple 1Ø permet d'obtenir après purification par chromatographie sur colonne, le produit sous forme d'une huile visqueuse incolore.

Rdt = 75 % CCM : Ø,7Ø-Ø,8Ø ; S.E.
Anal. (C23H19N) C, H, N
RMN : identique au produit de l'exemple 1Ø.

Chlorhydrate :

Rdt = 78 % F = 177°C (acet. éthyle)
$[\alpha]^{25}_{D}$ = - 49,5° (c = 3,2 ; méthanol)
Anal. (C23H29N, HCl) C, H, Cl, N

**Exemple 12 : α-cinnamyl-N-cyclobutylméthyl-α-éthyl-N-méthyl-benzylamine.**

(III ; R1 = R5 = C6H5 ; R2 = C2H5 ; R3 = CH3 ; m = 1 ; R4 = (CH2)CH)

Préparé selon le mode opératoire de l'exemple 1Ø précédent à partir de 15,Ø g (Ø,Ø56 mol) d'α-cinnamyl-α-éthyl-N-méthyl-benzylamine (intermédiaire F.3) et de 8,Ø5g (Ø,Ø68 mol) de chlorure d'acide cyclobutane carboxylique il est obtenu après purification par chromatographie sur colonne 18,Ø g (Rdt 92,5 %) de l'intermédiaire N-méthyl-N-cyclobutyl-carboxamide correspondant sous forme d'huile visqueuse (CCM : Ø,25-Ø,35 ; S.G.).

Le produit 16,5 g (Ø,Ø475 mol) est réduit tel que décrit à l'exemple 1Ø. Il est obtenu après purification par chromatographie sur colonne sous forme d'une huile visqueuse.

Poids : 8,5 g Rdt : 53,7 %
CCM : Ø,4Ø-Ø,6Ø ; S.H.
RMN : Ø,7Ø (t,3H); 1,4Ø-2,1Ø (m, 9H); 2,25 (s, 3H); 2,5Ø (s,2H); 2,85 (d,2H); 6,ØØ-6,6Ø (m,2H); 7,1Ø-7,6Ø (m,1ØH)

**Exemple 13 : α-cinnamyl-N-cyclopropylméthyl-N-α-diméthyl-benzylamine.**

(I.3 ; R1 = R5 = C6H5 ; R2 = R3 = CH3 ; m = 1 ; R4 = CH (CH2)2

Tel que décrit à l'exemple 1Ø, le composé est préparé à partir de l'intermédiaire décrit en F.4 et de chlorure de l'acide cyclopropanecarboxylique.

Rdt = 56 % (chromatographie) CCM : Ø,4Ø ; S.C.
RMN : Ø,ØØ-Ø,1Ø (m, 2H) ; Ø,2Ø-Ø,55 (m, 2H) ; Ø,55-1,ØØ (m, 1H) ; 1,3Ø (s, 3H) 1,85-2,25 (m, 2H) ; 2,4Ø (s, 3H) ; 2,5Ø-2,8Ø (m,2H ; 5,5Ø-6,35 (m, 2H) ; 7,1Ø-7,7Ø (m,1ØH)

**Exemple 14 : α-cinnamyl-N-cyclopropylméthyl-α-isopropyl-N-méthyl-benzylamine.**

(III ; R1 = R5 = C6H5 ; R2 = (CH3)2CH ; R3 = CH3 ; m = 1 ; R4 = CH(CH2)2

Selon l'exemple 1Ø et préparé à partir de l'intermédiaire décrit en F.5 et du chlorure de l'acide cyclopropanecarboxylique

Rdt = 78 % (chromatographie) CCM : 5Ø,35 ; S.C.
RMN : Ø,ØØ-Ø,1Ø (m, 2H) ; Ø,3Ø-Ø,55 (m, 2H) ; Ø,6Ø-Ø,9Ø (m, 7H) ; 2,1Ø-2,6Ø (m, 6H) ; 2,9Ø-3,2Ø (m,2H) ; 6,3Ø-6,7Ø (m, 2H) ; 7,1Ø-7,7Ø (m,1ØH)

**Exemple 15 : α-(3',4'-dichloro)cinnamyl-N-cyclopropylméthyl-α-éthyl-N-méthyl-p.méthoxybenzylamine**

(II ; R1 = p. CH3Ø-C6H4 ; R2 = C2H5; R3 = CH3; m = 1 ; R4 = CH(CH2)2 ; R5 = 3,4 C12-C6H3)

Selon l'exemple 1Ø et à partir de l'intermédiaire F.6 et du chlorure de l'acide cyclopropanecarboxylique

Rdt = 91 % (chromatographie) CCM : Ø,6Ø ; S.L.
RMN : Ø,ØØ-Ø,1Ø (m, 2H) ; Ø,3Ø-Ø,6Ø (m, 2H) ; Ø,6Ø-1,ØØ (m, 4H) ; 1,6Ø-2,ØØ (m, 2H) ; 2,2Ø-2,5Ø (m, 5H) ; 2,8Ø (d, 2H) ; 3,8Ø (s, 3H) ; 6,ØØ-6,5Ø (m, 2H) ; 6,7Ø-7,5Ø (m, 7H)

**Exemple 16 : α-(3',4'-dichloro)cinnamyl-N-cyclopropylméthyl-α-éthyl-N-méthyl-p.chlorobenzylamine**

(II ; R1 = p. Cl-C6H4 ; R2 = C2H5; R3 = CH3; m = 1 ; R4 = CH(CH2)2 ; R5 = 3,4 C12-C6H3)

Selon l'exemple 1Ø et à partir de l'intermédiaire F.7 et du chlorure de l'acide cyclopropanecarboxylique

Rdt = 57 % (chromatographie) CCM : Ø,45 ; S.K.

RMN : Ø,ØØ-Ø,15 (m, 2H) ; Ø,3Ø-Ø,6Ø (m, 2H) ; Ø,6Ø-1,ØØ (m, 4H) ; 1,55-2,Ø5 (m, 2H) ; 2,2Ø-2,5Ø (m, 5H) ; 2,8Ø (d, 2H) ; 5,9Ø-6,5Ø (m, 2H) ; 6,9Ø-7,5Ø (m, 7H)

**Exemple 17 : α-(3′,4′-dichloro)cinnamyl-N-cyclopropylméthyl-α-éthyl-N-méthyl-m.trifluorométhyl benzylamine**

(III ; R1 = m. F3C-C6H4 ; R2 = C2H5; R3 = CH3; m = 1 ; R4 = CH(CH2)2 ; R5 = 3,4 Cl2-C6H3)
Selon l'exemple 1Ø et à partir de l'intermédiaire F.8 et du chlorure de l'acide cyclopropanecarboxylique
Rdt = 64 % F = 66°C (hexanes) CCM : Ø,7Ø ; S.K.
RMN : Ø,ØØ-Ø,15 (m, 2H) ; Ø,3Ø-Ø,6Ø (m, 2H) ; Ø,6Ø-Ø,9Ø (m, 4H) ; 1,7Ø-2,1Ø (m, 2H) ; 2,35 (d, 2H) ; 2,5Ø (s, 3H) 2,85 (d, 2H) ; 5,9Ø-6,5Ø (m, 2H) ; 7,ØØ-7,9Ø (m, 7H)

**Exemple 18 : N-cyclopropylméthyl-α-éthyl-N-méthyl-α-(3′,4′,5′-triméthoxy)cinnamyl-benzylamine**

(III ; R1 = C6H5 ; R2 = C2H5; R3 = CH3; m = 1 ; R4 = CH (CH2)2 ; R5 = 3,4,5 (CH3Ø)3-C6H2)

Dans un réacteur protégé de l'humidité et sous atmosphère d'azote, on introduit 76,Ø ml d'une solution éthérée de bromure d'éthyl magnésium 3M (227 mmol).

On ajoute ensuite à une température comprise entre 2Ø et 3Ø°C, 2Ø,5 g (5Ø,4 mmol) d'amino nitrile IX décrit à la préparation G.1, en solution dans 65 ml de THF déshydraté sur tamis moléculaire.

Le mélange est agité 3 heures à la température ambiante puis introduit sans dépasser 1Ø°C dans 26Ø ml d'une solution aqueuse saturée en chlorure d'ammonium.

La phase aqueuse est écartée et la phase organique extraite 3 fois par une solution 2N d'acide chlorhydrique.

Les phases chlorhydriques réunies sont alcalinisées par une solution concentrée d'hydroxyde de sodium puis extraites à l'éther. Les phases éthérées sont réunies, lavées à l'eau puis déshydratées sur Na2SO4.

Après élimination de l'éther par distillation, le produit est purifié par chromatographie.
Poids : 11,Ø g Rdt = 53 % CCM : Ø,4Ø ; S.L.
RMN : Ø,ØØ-Ø,1Ø (m, 2H) ; Ø,3Ø-Ø,6Ø (m, 2H) ; Ø,6Ø-Ø,9Ø (m, 4H) ; 1,7Ø-2,1Ø (m, 2H) ; 2,3Ø-2,5Ø (m, 5H) ; 2,85 (d, 2H) ; 4,85 (S, 9H) ; 5,9Ø-6,55 (m, 2H) ; 6,6Ø (t, 2H); 7,1Ø-7,6Ø (m, 5H)

**Exemple 19 : α-cinnamyl-N-cyclopropylméthyl-α-éthyl-N-méthyl-α-3,4,5-triméthoxy)benzylamine**

(III ; R1 = 3,4,5(CH3Ø)3-C6H2 ; R2 = C2H5; R3 = CH3; m=1; R4 = CH (CH2)2 ; R5 = C6H5)

Le composé est préparé selon le mode opéatoire de l'exemple précédent par réaction de bromure d'éthyl magnésium sur l'α-amino-α-cinnamyl-N-cylcopropylméthyl-N-méthyl-(3,4,5-triméthoxy)phénylacétonitrile
Rdt = 47 % Eb/Ø,Ø25 = 175°C CCM : Ø,3Ø ; S.C.
RMN : Ø,2Ø-Ø,6Ø (m, 4H) ; Ø,9Ø (m, 4H) ; 1,9Ø (q, 2H) ; 2,4Ø (m, 2H) ; 2,5Ø (s, 3H) ; 2,8Ø (d, 2H) ; 3,9Ø-4,ØØ (m, 9H) ; 6,3Ø-6,7Ø (m, 2H) ; 6,85 (s, 2H) ; 7,2Ø-7,4Ø (M, 5H)
Anal. (C26H35NO3) C, H, N, O

Les essais toxicologiques et pharmacologiques réalisés avec les composés des exemples 1 à 19 précèdents mettent en évidence leur faible toxicité de même que, chez la souris, la capacité d'inhiber les convulsions induites par la picrotoxine.

Cette propriété permet d'envisager une utilité des produits de l'invention comme psychotropes dans le traitement des affections neuropsychiques.

Par ailleurs, et dans le but de poursuivre l'étude de leurs propriétés pharmacologiques, les affinités de liaison "in vitro" aux récepteurs mu, delta, kappa ont été étudiées, de même que l'affinité de liaison aux récepteurs sigma.

Les résultats des études effectuées montrent pour les composés de l'invention une affinité de liaison particulière pour les récepteurs sigma et, de façon plus manifeste lorsque dans les composés R1 et R5 sont phényle.

Cette propriété permet d'envisager pour les composés de l'invention une activité anti psychotique, la corrélation ayant été proposée récemment par Brian L. Largent et coll. (Eur. J. of Pharmacol. 155 (1988) p. 345-347) qui constatent que des composés divers de par leur structure mais tous potentiellement anti-psychotiques ont une propriété commune qui consiste en une affinité "in vitro" aux récepteurs sigma.

Egalement, mais "in vivo" chez le rat, les benzylamines (I) antagonisent les phénomènes amnésiques provoqués par la scopolamine et inhibent au niveau gastro-duodénal les ulcères provoqués par administration de cystéamine. Cette activité est reliée avec la propriété des produits à augmenter la sécrétion duodénale alcaline chez l'animal anesthésié. L'ensemble de ces propriétés, concrétisé par les études effectuées et leurs résultats

présentés dans le présent mémoire rendent les composés selon l'invention utiles à la prévention et au traitement des asthénies et des désordres d'ordre neurologique et/ou psychique de même qu'au traitement de divers dysfonctionnements du tractus gastro-intestinal.

Les études démonstratives des propriétés des produits de l'invention et les résultats obtenus sont rapportés ci-après :

a) la toxicité des produits de l'invention est recherchée chez la souris par la détermination approchée de leur DL 5Ø, qui est la dose léthale provoquant 5Ø % de morts chez les animaux dans les conditions de l'expérience. L'étude est réalisée sur des lots de quatre souris "Swiss" mâles d'un poids de 2Øg environ mises à jeun la veille de l'étude.

Chaque détermination est effectuée avec quatre doses correspondant respectivement à une administration par voie orale de 1ØØ, 3ØØ, 6ØØ et 1ØØØ mg de produit, exprimées sous forme de base, par kg d'animal.

Il est constaté par cette étude que les produits de l'invention ont une toxicité aigüe correspondant à une DL5Ø supérieure ou égale à 1.ØØØ mg/kg. Exceptionnellement, cette toxicité peut-être d'environ 6ØØ mg/kg.

b) Les propriétés psychotropes des composés ont été déterminées par la protection des convulsions induites par la picrotoxine chez la souris, qui a été réalisée selon une méthode dérivée de celle de Krall et coll., "Epilepsia", 1978, 19, p.4Ø9-428.

L'administration de picrotoxine provoque chez l'animal une crise convulsive caractérisée par un syndrome d'extension myoclonique suivi de l'extension des membres conduisant à la mort de l'animal. Certaines substances, notamment celles actives sur le complexe GABA/benzodiazépines/Cl-ionophore permettent de protéger les animaux de cette crise convulsive.

Pratiquement l'étude est réalisée sur des lots de 1Ø souris "Swiss" mâles, d'un poids de 2Ø g environ auxquelles on administre le produit à étudier en solution aqueuse soit par voie intrapéritonéale (i.p.) soit par voie orale (p.o).

On pratique ensuite une injection par voie intrapéritonéale d'une solution de picrotoxine à raison de 24 mg/kg sous un volume de Ø,2 mlpar animal, soit 3Ø minutes après l'administration du produit par voie intrapéritonéale, soit 6Ø minutes après l'administration du produit par voie orale, la dose de produit ainsi injectée provoquant une crise clonique qui conduit à la mort des animaux non traités. Dans les conditions du test, on observe chez les animaux traités la suppression de la phase tonique d'extension.

Les résultats sont exprimés :

- soit en pourcentage d'animaux protégés de cette phase sous l'action de 5Ø mg/kg du composé à l'étude administré par voie i.p. ou 1ØØ mg/kg par voie p.o.,
- soit en DE5Ø pour chacune de ces voies, qui est la dose efficace de composé à l'essai exprimée en mg/kg, protégeant 5Ø % des animaux de cette phase d'extension, la valeur significative des résultats étant généralement indiquée de la façon suivante :

```
*        Résultat significatif à p. < Ø,Ø5
**          "           "        à p. < Ø,Ø1
***         "     hautement significatif à p. < Ø,ØØ1
```

Les résultats de l'étude sont reportés pour les produits de l'invention II et III de formule générale I dans le tableau 1 qui suit et montrent pour les deux voies d'administration l'activité protectrice des composés de l'invention étudiés.

Tableau 1 : Inhibition des convulsions induites par la picrotoxine

| Exemple | i.p. : % prot. ou DE50 | p.o. : % prot. ou DE50 |
|---|---|---|
| 2 | NT | 90 % *** |
| 8 | NT | DE50 = 53,6 |
| 9 | 90 % *** | NT |
| 10 | 50 % * | NT |
| 11 | 70 % ** | NT |
| 13 | DE50 = 32,5 | DE50 = 64,4 |
| 16 | NT | 60 % ** |

N.T. Non testé

c) l'étude "in vitro" de l'affinité des composés aux récepteurs sigma est réalisée selon la technique décrite par Largent B.L. et coll. dans J. Pharmacol. Exp. Thér. 238, 1986, p. 739-748 dont le principe est de mettre en compétition les affinités respectives du produit à étudier et celle du (+) [3H] SKF 10,047 qui est le ligand radioactif caractéristique des récepteurs sigma des membranes de cerveau de cobaye qui l'on utilise dans cette étude.

L'essai est réalisé en faisant incuber des solutions de concentrations appropriées des produits avec des prélèvements standards de membranes puis à déterminer après filtration, la radioactivité résiduelle de la solution.

A l'aide d'un système informatique équipé d'un logiciel adapté, les résultats sont traités de façon à calculer la CI50 du produit à l'étude qui est, dans ce cas, la concentration nanomolaire de solution capable d'inhiber 50 % des liaisons du ligand radioactif aux récepteurs sigma des membranes utilisées.

Les résultats sont présentés au tableau 2 qui suit et, à titre de référence, il est aussi présenté celui obtenu avec la Ditolylguanidine (DTG) qui est considérée comme un ligand sélectif et de grande affinité aux récepteurs sigma (Stephen G. Holtzman. J. Pharm. Exp. Ther. Vol. 248, n° 3, 1989, p. 1054-1062), toutefois n'est uniquement utilisé que comme réactif pharmacologique.

Tableau 2 : Affinité de liaison des composés de l'invention aux récepteurs sigma

| Composés à l'essai Exemple | CI50 (nmol) |
|---|---|
| 3 | 368 |
| 6 | 246 |
| 7 | 32 |
| 8 | 21 |
| 9 | 24 |
| 10 | 13 |
| 11 | 102 |
| 12 | 50 |
| DTG | 103 |

Ces résultats montrent que les benzylamines suivant l'invention ont une affinité aux récepteurs sigma du même ordre de grandeur et parfois près de 1∅ fois supérieure à celle de la DTG sur ces mêmes récepteurs.

Ces affinités, associées aux faibles toxicités des benzylamines (I) sont démonstratives de leur supériorité comparées au DTG.

La spécificité d'affinité des composés pour les récepteurs sigma est montrée par l'étude comparée de l'affinité des produits aux récepteurs opiacés mu, delta et kappa, ainsi qu'aux récepteurs de la phencyclidine (PCP) qui sont des récepteurs reconnus être impliqués comme médiateurs dans l'effet de drogues psychomimétiques (Eric J. Simon - "Opiates receptor binding in Drug Research" p. 183-199 dans "Receptor Binding in Drug Research" - Ed. Robert A. O'Brien-Marcel Dekker - 1986 et également Brian L. Largent et coll. dans Eur. J. of Pharmacol. 155 (1988) p. 345-347).

L'étude "in vitro" de l'affinité des composés de l'invention aux trois récepteurs opiacés est réalisée selon la technique décrite par F. Roman et coll. dans J. Pharm. Pharmacol. 1987, 39, p. 4∅4-4∅7 et l'étude de l'affinité aux récepteurs de la PCP est réalisée selon la technique décrite par Vignon J. et coll. dans "Brain Res." 1983 ; 28∅, p. 194-7 et 1986 ; 378, p. 133-41.

Les résultats présentés au tableau 3 sont pour chaque récepteur étudié, exprimés en CI5∅ qui sont les concentrations nanomolaires des produits en solutions capables d'inhiber 5∅ % des liaisons du ligand radioactif spécifique lié au récepteur considéré.

Tableau 3 : Affinité des produits de l'invention aux récepteurs sigma comparée à leurs affinités aux récepteurs mu, delta, kappa et PCP

| Composés à l'essai Exemple | Rec. mu | Rec. delta | Rec. Kappa | Rec. sigma | Rec. PCP |
|---|---|---|---|---|---|
| 7 | > 99999 | 51750 | 72800 | 22 | 50800 |
| 8 | 2040 | 7550 | 1520 | 21 | 25900 |
| 9 | 15300 | 33900 | 6200 | 24 | 3670 |
| 10 | 6320 | 67900 | 23800 | 13 | 4590 |
| 11 | 6580 | 41600 | 5140 | 210 | 7100 |
| 12 | 7190 | 35600 | 6280 | 50 | NT |
| 13 | 10200 | 9370 | 8680 | 337 | 10500 |
| 14 | 34200 | > 99999 | 76000 | 569 | 14150 |
| DTG | 3970 | 33200 | 6490 | 103 | 6750 |

N.T. : non testé

Une spécificité d'affinité sigma est évidente pour les composés préférés de l'invention.

Ainsi, si l'on attribue la valeur 1 pour la CI5Ø d'affinité sigma, les valeurs comparées des CI5Ø aux autres récepteurs sont calculés pour les composés des exemples 7, 8, 9, 1Ø et 12 dont l'affinité sigma est la plus intense. Ces valeurs sont comparées à celles obtenues avec le DTG présenté comme composé de référence.

| Composés à l'essai Exemple | Rec. mu | Rec. delta | Rec. Kappa | Rec. sigma | Rec. PCP |
|---|---|---|---|---|---|
| 7 | >1000 | 1617 | 2275 | 1 | 1597 |
| 8 | 97 | 359 | 72 | 1 | 1233 |
| 9 | 658 | 3491 | 258 | 1 | 153 |
| 10 | 486 | 5223 | 1930 | 1 | 353 |
| 12 | 143 | 1712 | 125 | 1 | - |
| DTG | 38 | 322 | 63 | 1 | 65 |

Ce mode d'expression montre que, dans les conditions opératoires décrites, les composés préférés de l'invention ont une affinité pour les récepteurs sigma environ 1ØØ fois supérieure à celle déterminée pour les autres récepteurs étudiés et dans plusieurs cas plus de 1ØØØ fois supérieure.

Comparés aux produits de l'invention, la ditolylguanide (DTG) composé de référence annoncé sélectif des récepteurs sigma, montre des valeurs parfois voisines mais toujours inférieures et quelque soit le récepteur considéré aux valeurs calculées pour les produits préférés de l'invention.

Cette expression particulière de l'étude illustre la sélectivité d'affinité remarquable des produits de l'invention aux récepteurs sigma.

d) l'aptitude des benzylamines (I) à antagoniser chez l'animal l'amnésie provoquée par la scopolamine est mise en évidence par le test d'évitement passif qui est réalisé chez la souris selon une méthode décrite par Lenègre et coll. ; il consiste dans son principe à utiliser une boîte à deux compartiments dont le plus petit est éclairé et le plus grand qui est obscur est équipé d'un système permettant de délivrer un courant électrique de faible intensité par le plancher de l'appareil.

L'animal est introduit dans le compartiment éclairé et lorqu'il se déplace dans le compartiment obscur, il reçoit par le socle un courant électrique (Ø,35 mA) jusqu'à ce qu'il retourne dans le compartiment éclairé d'où il est alors enlevé.

Après 24 heures l'opération est répétée. La souris est replacée dans l'appareil, elle évite de pénétrer dans le compartiment obscur. Le temps d'attente avant le passage dans ce compartiment est mesuré ; il permet dans les conditions de l'expérience d'apprécier l'efficacité des composés à l'essai à antagoniser l'effet amnésique de la scopolamine qui est administré par voie intrapéritonéale à raison de 1 mg/kg 3Ø minutes avant la première introduction de l'animal dans l'appareil.

Chez les souris témoins n'ayant reçu que la scopolamine, la durée d'attente du passage après 24 heures est remarquablement plus faible que celle observée chez les animaux non traités. Chez les animaux ayant reçu à la fois la scopolamine et le produit à l'essai cette durée est notablement allongée sous l'action anti amnésique des composés actifs.

L'étude des produits de l'invention selon cette méthode est réalisée sur des lots de 18 souris, les produits sont administrés au premier jour par voie orale à raison de Ø,25 mg/kg 3Ø minutes avant l'administration de la scopolamine par voie i.p..

Les résultats sont présentés au tableau 4 et sont exprimés en pourcentage d'effet antagoniste à l'amnésie provoquée par la scopolamine, l'antagonisme étant déterminé à partir des temps de latence montrés par les animaux dans le second essai après 24 heures pour pénétrer dans le compartiment obscur.

Tableau 4 : Activité des produits de l'invention sur l'amnésie provoquée chez le rat par administration de scopolamine

| Composés à l'essai (0,25 mg/kg) | % antagonisme à l'amnésie |
|---|---|
| 7 | 56 % |
| 9 | 76 % |
| 10 | 76 % |
| 11 | 38 % |
| 12 | 98 % |
| PIRACETAM (2048 mg/kg) | 109 % |

Ces résultats sont probants d'une activité des produits de l'invention à faible dose et notamment des composés des exemples 9, 1∅ et 12.

Dans ce test, la substance standard de référence qui fait preuve de la bonne marche de l'essai est le Piracetam. Il provoque à raison de 2∅48 mg/kg po qui est une dose pharmocologique considérable et inapplicable à des fins pharmaceutiques, un antagonisme à l'amnésie d'une valeur d'environ 1∅∅ %.

e) L'activité des composés de l'invention sur le tractus gastrointestinal a été montrée chez le rat par leur aptitude à inhiber les ulcères gastroduodénaux provoqués par administration de cystéamine. Cette activité est mise en relation avec la capacité des produits à provoquer une augmentation de la sécrétion alcaline au niveau du duodénum chez l'animal anesthésié.

- l'activité inhibitrice sur les ulcère gastro duodénaux provoqués chez le rat par la cystéamine a été mise en évidence selon une technique décrite par Robert et coll., dans "Digestion", 1974, 11, p. 199-211.

Dans cet essai, des lots de 6 rats Wistar femelle d'un poids moyen de 2∅∅ gr reçoivent une injection sous cutanée de chlorhydrate de cystéamine à raison de 4∅∅ mg/kg. Les produits à tester sont administrés par voie orale ou par voie sous cutanée respectivement 1 h ou 3∅ minutes avant l'agent ulcérogène

Dix huit heures après, les rats sont sacrifiés par élongation, leur estomac et duodénum prélevés, rincés avec du soluté physiologique et épinglés sur un carton. La présence d'ulcères de la zone antro-pylor-duodénale est recherchée et leur surface, exprimée en mm2, est évaluée en multipliant les deux axes perpendiculaires principaux de la lésion. L'analyse statistique des résultats est effectuée à l'aide du test de Student pour les surfaces ulcèrées, comparativement à un groupe contrôle ne recevant que l'excipient.

Les résultats présentés au tableau 5, sont exprimés en DE5∅ de scores d'ulcération qui sont les doses efficaces exprimées en mg/kg de produit qui provoquent l'inhibition de 5∅ % des ulcérations provoquées par la cystéamine.

Tableau 5 : Activité inhibitrice sur les ulcères gastro-duodénaux provoqués par la cystéamine

| Produit à l'essai Exemple | DE50 - Scores d'ulcérations mg/kg |
|---|---|
| 7 | 10,5 |
| 9 | 26,2 |
| 10 | 5,0 |
| 12 | 12,2 |

- l'activité des produits sur la sécrétion alcaline duodénale chez le rat, est étudiée selon une technique décrite par FLEMSTROM et coll. Gastroenterolgy, 1983, 84, p. 787-794).

L'essai consiste à déterminer toutes les 1Ø minutes et durant 3 heures, in situ, la sécrétion alcaline d'un segment de duodénum d'une longueur de 12 mm, exempt de glandes de Brunner et situé à 2 cm du pylore.

L'étude est réalisée chez des rats mâles de 35Ø g environ qui sont préalablement anesthésiés et pour lesquels le segment de duodénum choisi est cannulé par 2 tubes de verre selon la technique décrite par l'auteur, et dans lequel un perfusat luminal constitué par 5 ml de solution isotonique de NaCl est maintenu à 37°C.

Les variations de sécrétion alcaline intraluminale sont mesurées par pH métrie toutes les 1Ø minutes au moyen d'un ensemble automatisé maintenant un pH constant de 7,4, par addition in situ de solution d'acide chlorhydrique Ø,Ø4 N. Cinquante minutes après le début de l'expérience, les produits à l'essai sont administrés par voie intraveineuse ou intraartérielle à raison de 1 mg/kg et le volume d'acide chlorhydrique introduit, qui est le reflet de l'augmentation in situ de la sécrétion alcaline, est mesuré toutes les 1Ø minutes durant 3 heures par rapport au début de l'expérimentation soit pendant 13Ø minutes après l'injection du produit à l'essai.

Pour chaque produit il est déterminé :

a) l'augmentation de sécrétion maximale en micro Eq/cm/n sous l'action de la drogue et compte tenu de la sécrétion basale des mêmes animaux observée durant 5Ø minutes avant l'administration du produit.

b) l'augmentation de l'aire sous la courbe (AUC) comprise dans l'intervalle 1 h à 2 h de l'expérience sur le débit alcalin des animaux traités comparée à celle des animaux témoins.

Ces augmentations étant représentatives de l'activité à favoriser la sécrétion alcaline.

Les résultats sont présentés au tableau 6 qui suit pour les produits (I) de l'invention administrés par voie intraveineuse à raison de 1 mg/kg.

TABLEAU 6 : Activite des produits de l'invention sur la sécrétion alcaline duodenale chez le rat anesthésié.

| Produit à l'essai Exemple | Augmentation sécrétion micro Eq/cm/h | Augmentation AUC 1 2 h |
|---|---|---|
| 7 | 6,0 | 52,8 |
| 9 | 10,0 | 97,6 |
| 10 | 7,5 | 66,2 |
| 12 | 7,0 | 41,6 |

Ces essais, réalisés au niveau gastroduodénal chez le rat montrent que les produits de l'invention ont une activité probante à inhiber les ulcères tels que ceux provoqués par la cystéamine. Il est constaté une relation probable entre cette activité inhibitrice et la propriété des mêmes produits de l'invention à augmenter la sécrétion alcaline duodénale.

Ces propriétés pharmacologiques, associées à la faible toxicité des composés de l'invention permettent d'envisager leur utilité, sous forme de médicaments aux traitements préventifs et curatifs d'affections d'ordre neurologique et/ou psychique en général, comme, par exemples, les états dépressifs, les troubles de la mémoire et/ou du comportement, la schizophrénie, la maladie d'Alzheimer, la maladie de Parkinson et la démence sénile.

Egalement les benzylamines (I) sont appropriées au traitement des dysfonctionnements du tractus gastrointestinal en général comme par exemple les troubles du péristaltisme, de la motricité, les phénomènes de reflux gastro oesophagiens et gastroduodénaux ainsi que les ulcérations gastriques et gastroduodénales.

De façon habituelle les doses unitaires utiles sont comprises entre 1 et 5ØØ mg et plus particulièrement entre 5 et 2ØØ mg de produit selon la nature et la gravité de l'affection a traiter. Les doses thérapeutiques journalières peuvent être réparties en plusieurs prises et sont comprises entre 5 et 2ØØØ mg de produit par jour. De façon générale une posologie journalière de 5Ø à 5ØØ mg de produit par jour répartis en deux à quatre prises est satisfaisante.

L'administration des produits de l'invention aux patients à traiter est réalisée sous la forme de médicaments de nature adaptée à l'affection à soigner. Selon les cas les préparations médicamenteuses seront, comme exemples non limitatifs, des comprimés, dragées, capsules, poudres, solutions, suspensions, gels ou suppositoires. Ces formes pharmaceutiques sont préparées à partir des produits sous forme de base ou de leurs sels et selon des méthodes couramment pratiquées dans cette industrie.

Généralement dans les formes médicamenteuses de nature solide, le principe actif représente de 5 à 9Ø % en poids du total de la forme terminée alors que les excipients représentent de 95 à 1Ø %. Pour les formes liquides, ou pouvant être considérées comme telles, la quantité de principe actif est comprise entre Ø,1 et 1Ø % en poids de la forme terminée alors que les excipients peuvent représenter de 99,9 à 9Ø % en poids de cette forme.

A titre d'exemple il est présenté la formulation et la préparation des solutés isotoniques injectables, celle de comprimés et celle de gels administrables par voie orale.

Soluté isotonique injectable

- Formule :

| | |
|---|---|
| Substance active de l'exemple 10 (chlorhydrate) | 10 mg |
| Chlorure de sodium | 9 mg |
| Eau distillée en quantité suffisante pour | 1,0 ml |

- Préparation :

Le soluté isotonique est réparti en ampoules de volume approprié qui sont, après scellement, stérilisée par des moyens thermiques connus en soi, ou bien le soluté est stérilisé par filtration, réparti en ampoules qui sont ensuite scellées, l'ensemble des opérations étant effectué sous atmosphère stérile.
Dans ce dernier cas, on préfère ajouter à la formule décrite, 1% d'alcool benzylique à titre d'agent bactériostatique, soit 10 mg de cet alcool par ml de soluté.

Comprimés

- Formule

| | |
|---|---|
| Substance active de l'exemple 9 (hémimaléate) | 10,0 à 50,0 mg |
| Polyvinylpyrrolidone | 20,0 mg |
| Carboxyméthylamidon | 8,0 mg |
| Stéarate de magnésium | 2,0 mg |
| Silice colloidale | 0,4 mg |
| Lactose en quantité suffisante pour | 200,0 mg |

- Préparation

Le principe actif est mélangé au lactose puis granulé avec la polyvinylpyrrolidone en solution. Les grains sont séchés et tamisés sur une grille d'ouverture de 1 mm. Le carboxyméthylamidon est mélangé à la silice colloidale puis ajouté aux granulés. On mélange ensuite intimement avec le stéarate de magnésium puis comprime à raison de 200,0 mg par comprimé.

Gel

- Formule

| | |
|---|---|
| Substance active de l'exemple 9 (hémimaléate) | 0,20 à 0,60 g |
| Hydroxypropylcellulose | 2,00 g |
| Saccharinate de sodium | 0,01 g |
| Sirop de sorbitol à 70% (p/v) | 25,00 g |
| Arome naturel de fraise | 0,50 g |
| Conservateur | 0,10 g |
| Eau purifiée en quantité suffisante pour | 100,00 g |

-Préparation :

Les conservateurs et le saccharinate de sodium sont dissous dans l'eau, puis, sous agitation, on ajoute en la dispersant l'hydroxypropylcellulose. L'agitation est maintenue jusqu'à obtention d'un gel auquel, toujours sous agitation, on ajoute le sirop de sorbitol puis finalement l'arome.

## Revendications

### Revendications pour les Etats contractants suivants : AT, BE, CH, DE, FR, GB, IT, LI, LU, NL, SE

**1.** N-cycloalkylalkyle benzylamines disubstituées, de formule I :

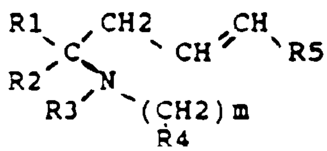

I

dans laquelle:

- R1 est phényle éventuellement mono, di ou trisubstitué par des atomes d'halogène par des radicaux alcoyle inférieurs, par des radicaux haloalkyle inférieurs ou alkoxy inférieurs,
- R2 est alkyle inférieur,
- R3 est l'hydrogène ou aklyle inférieur,
- R4 est cycloalkyle -CH(CH2)n, dans lequel n a pour valeur un entier de 2 à 5, un atome de carbone de R4 pouvant porter un radical Rx qui est alkyle inférieur ou phényle,
- R5 est phényle éventuellement mono, di ou trisubstitué par des atomes d'halogène par ou des radicaux alkoxy inférieurs.
- m a pour valeur 1 ou 2, leurs sels d'addition avec les acides et leurs formes optiquement actives.

**2.** N-cycloalkylalkyle benzylamines suivant la revendication 1, caractérisées en ce que R1 est phényle.

**3.** N-cycloalkylalkyle benzylamines suivant les revendications 1 ou 2 caractérisées en ce que R5 est phényle.

**4.** N-cycloalkylalkyle benzylamines suivant les revendications 1 à 3 caractérisées en ce que R2 est éthyle.

**5.** N-cycloalkylalkyle benzylamines suivant les revendications 1 à 4 caractérisées en ce que R3 est l'hydrogène ou méthyle.

**6.** N-cycloalkylalkyle benzylamines suivant l'une des revendications 1 à 5 caractérisées en ce que Rx est un radical méthyle ou phényle.

**7.** Procédé de préparation des N-cycloalkylalkyle benzylamines de formule (I) caractérisé en ce qu'il consiste :

- pour obtenir une benzylamine de formule (II) répondant à la formule (I) dans laquelle R3 est l'hydrogène :

i) à acyler une benzylamine (V) de formule :

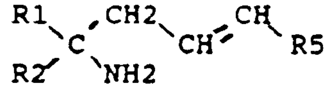

par un agent d'acylation (R4-[CH2]m-1]-CO)pZ1 (VI) dans lequel Z1 est un radical hydroxyle ou un atome de chlore ou de brome lorsque p = 1, ou Z1 est un atome doxygène quand p=2, pour obtenir un intermédiaire N-carboxamide (IV)

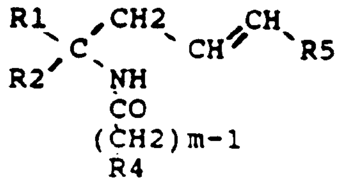

puis à le réduire par un hydrure métallique
ii) à alkyler une benzylamine (V) précédemment décrite par une halogénure de cycloalkylalkyle R4-(CH2)m-Z2 dans lequel Z2 est le chlore, le brome ou l'iode.
- pour obtenir une benzylamine de formule (III) répondant à la formule (I) dans laquelle R3 est alkyle inférieur,
i) à procéder à l'alkylation réductrice d'une benzylamine (I) de formule (II) de l'invention par un aldéhyde R6-CHO dans lequel R6 est l'homologue carboné inférieur au radical R3 (R3=CH2-R6) et un réducteur tel qu'un hydrure métallique ou organométallique,
ii) ou à acyler une benzylamine (VII) de formule

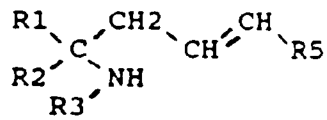

dans laquelle R3 est alkyle inférieur par un halogénure d'acyle R4-[(CH2)m-1]COZ4 dans lequel Z4 est le chlore ou le brome, pour obtenir un N-carboxamide intermédiaire (VIII)

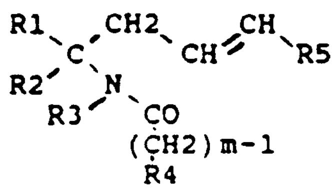

que l'on réduit par un hydrure métallique
iii) ou à faire réagir un réactif organomagnésien R2MgZ3 dans lequel Z3 est le chlore, le brome ou l'iode, sur un aminonitrile (IX) dans lequel R3 est alkyle inférieur.

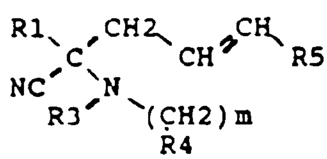

iiii) ou alkyler une benzylamine (VII) déjà décrite par un halogénure d'alkyle R'-(CH2)m-Z2 déjà décrit.
- pour obtenir un sel à salifier une benzylamine(I) par un acide et pour obtenir une forme optiquement active à résoudre un racémique.

**8.** Médicament, notamment psychotrope et utile aux traitements des psychoses caractérisé en ce qu'il comprend une N-cycloalkylalkyle benzylamine suivant l'une des revendications 1 a 6.

**9.** Médicament utile au traitement des affections gastro-intestinales caractérisé en ce qu'il comprend une N-cycloalkylalkyle benzylamine suivant l'une des revendications 1 à 6.

**10.** Composés intermédiaires de formule :

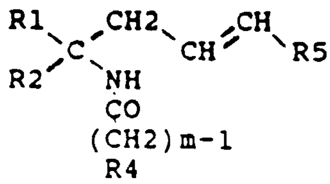

IV

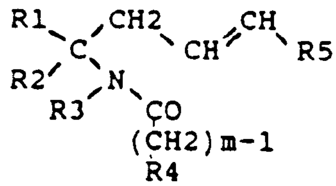

VIII

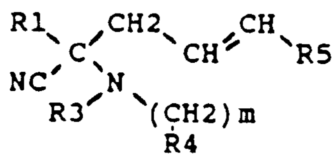

IX

dans lesquels R1, R2, R3, R4, R5 et m ont les significations désignées pour les benzylamines (I), R3 n'étant toutefois pas l'hydrogène.

**Revendications pour l'Etat contractant suivant : GR**

**1.** N-cycloalkylalkyle benzylamines disubstituées, de formule I :

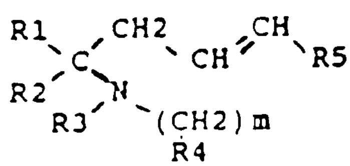

I

dans laquelle:
- R1 est phényle éventuellement mono, di ou trisubstitué par des atomes d'halogène par des radicaux alcoyle inférieurs, par des radicaux haloalkyle inférieurs ou alkoxy inférieurs,
- R2 est alkyle inférieur,
- R3 est l'hydrogène ou alkyle inférieur,
- R4 est cycloalkyle -CH(CH2)n, dans lequel n a pour valeur un entier de 2 à 5, un atome de carbone de R4 pouvant porter un radical Rx qui est alkyle inférieur ou phényle,
- R5 est phényle éventuellement mono, di ou trisubstitué par des atomes d'halogène par ou des radicaux alkoxy inférieurs.
- m a pour valeur 1 ou 2,

leurs sels d'addition avec les acides et leurs formes optiquement actives.

**2.** N-cycloalkylalkyle benzylamines suivant la revendication 1, caractérisées en ce que R1 est phényle.

**3.** N-cycloalkylalkyle benzylamines suivant les revendications 1 ou 2 caractérisées en ce que R5 est phényle.

**4.** N-cycloalkylalkyle benzylamines suivant les revendications 1 à 3 caractérisées en ce que R2 est éthyle.

**5.** N-cycloalkylalkyle benzylamines suivant les revendications 1 à 4 caractérisées en ce que R3 est l'hydrogéne ou méthyle.

6. N-cycloalkylalkyle benzylamines suivant l'une des revendications 1 à 5 caractérisées en ce que Rx est un radical méthyle ou phényle.

7. Procédé de préparation des N-cycloalkylalkyle benzylamines de formule (I) caractérisé en ce qu'il consiste :

- pour obtenir une benzylamine de formule (II) répondant à la formule (I) dans laquelle R3 est l'hydrogène :

i) à acyler une benzylamine (V) de formule :

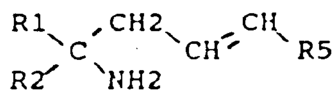

par un agent d'acylation (R4-[CH2)m-1]-CO)pZ1 (VI) dans lequel Z1 est un radical hydroxyle ou un atome de chlore ou de brome lorsque p = 1, ou Z1 est un atome doxygène quand p=2, pour obtenir un intermédiaire N-carboxamide (IV)

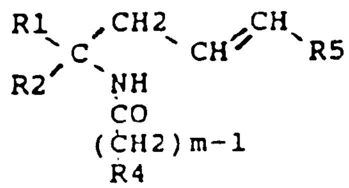

puis à le réduire par un hydrure métallique

ii) à alkyler une benzylamine (V) précédemment décrite par un halogénure de cycloalkylalkyle R4-(CH2)m-Z2 dans lequel Z2 est le chlore, le brome ou l'iode.

- pour obtenir une benzylamine de formule (III) répondant à la formule (I) dans laquelle R3 est alkyle inférieur,

i) à procéder à l'alkylation réductrice d'une benzylamine (I) de formule (II) de l'invention par un aldéhyde R6-CHO dans lequel R6 est l'homologue carboné inférieur au radical R3 (R3=CH2-R6) et un réducteur tel qu'un hydrure métallique ou organométallique,

ii) ou à acyler une benzylamine (VII) de formule

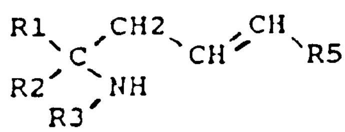

dans laquelle R3 est alkyle inférieur par un halogénure d'acyle R4-[(CH2)m-1]COZ4 dans lequel Z4 est le chlore ou le brome, pour obtenir un N-carboxamide intermédiaire (VIII)

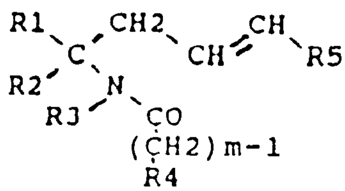

que l'on réduit par un hydrure métallique

iii) ou a faire réagir un réactif organomagnésien R2MgZ3 dans lequel Z3 est le chlore, le brome ou l'iode, sur un aminonitrile (IX) dans lequel R3 est alkyle inférieur.

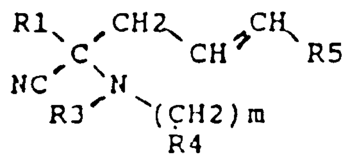

iiii) ou alkyler une benzylamine (VII) déjà décrite par un halogénure d'alkyle R'-(CH2)m-Z2 déjà décrit, pour obtenir un sel d'addition avec un acide à salifier la benzylamine par un acide et pour obtenir une forme optiquement active à dédoubler un racémique.

8. Procédé de préparation d'un médicament, notamment psychotrope et utile aux traitements des psychoses, caractérisé en ce qu'il consiste à mélanger une N-cycloalkylalkyle benzylamine suivant l'une des revendications 1 à 6 à un excipient pharmaceutique.

9. Procédé de préparation d'un médicament utile au traitement des affections gastro-intestinales, caractérisé en ce qu'il consiste à mélanger une N-cycloalkylalkyle benzylamine suivant l'une des revendications 1 à 6 à un excipient pharmaceutique.

10. L'utilisation d'une benzylamine telle que définie aux revendications 1 à 6 ou de l'un de ses sels acceptables thérapeutiquement pour l'obtention d'un médicament psychotrope et utile aux traitements des psychoses.

11. L'utilisation d'une benzylamine telle que définie aux revendications 1 à 6 ou de l'un de ses sels acceptables thérapeutiquement pour l'obtention d'un médicament utile au traitement des affections gastro-intestinales.

12. Composés intermédiaires de formule :

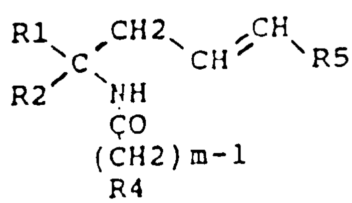

IV

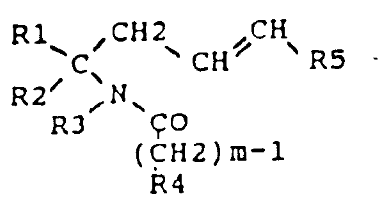

VIII

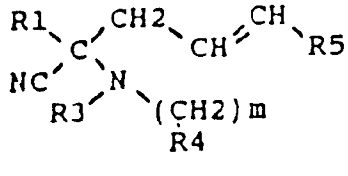

IX

dans lesquels R1, R1, R3, R4, R5 et m ont les significations désignées pour les benzylamines (I), R3 n'étant toutefois pas l'hydrogène.

**Revendications pour l'Etat contractant suivant : ES**

1. Procédé de préparation des N-cycloalkylalkyle benzylamines de formule (I) :

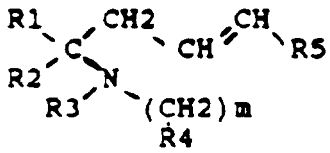

I

dans laquelle :

- R1 est phényle éventuellement mono, di ou trisubstitué par des atomes d'halogène par des radicaux alcoyle inférieurs, par des radicaux haloalkyle inférieurs ou alkoxy inférieurs;
- R2 est alkyle inférieur,
- R3 est l'hydrogène ou alkyle inférieur,
- R4 est cycloalkyle -CH(CH2)n, dans lequel n a pour valeur un entier de 2 à 5, un atome de carbone de R4 pouvant porter un radical Rx qui est alkyle inférieur ou phényle,
- R5 est phényle éventuellement mono, di ou trisubstitué par des atomes d'halogène par ou des radicaux alkoxy inférieurs,
- m a pour valeur 1 ou 2,

de leurs sels d'addition avec les acides et de leurs formes optiquement actives,

caractérisé en ce qu'il consiste :

- pour obtenir une benzylamine de formule (II) répondant à la formule (I) dans laquelle R3 est l'hydrogène :

i) à acyler une benzylamine (V) de formule :

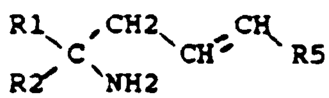

par un agent d'acylation (R4-[CH2]m-1]-CO)pZ1 (VI) dans lequel Z1 est un radical hydroxyle ou un atome de chlore ou de brome lorsque p = 1, ou Z1 est un atome d'oxygène quand p=2, pour obtenir un intermédiaire N-carboxamide (IV)

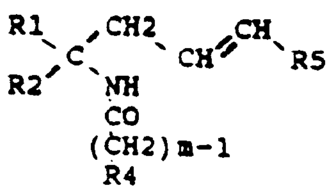

puis à le réduire par un hydrure métallique

ii) à alkyler une benzylamine (V) précédemment décrite par un halogénure de cycloalkylalkyle R4-(CH2)m-Z2 dans lequel Z2 est le chlore, le brome ou l'iode.

- pour obtenir une benzylamine de formule (III) répondant à la formule (I) dans laquelle R3 est alkyle inférieur,

i) à procéder à l'alkylation réductrice d'une benzylamine (I) de formule (II) de l'invention par un aldéhyde R6-CHO dans lequel R6 est l'homologue carboné inférieur au radical R3 (R3=CH2=R6) et un réducteur tel qu'un hydrure métallique ou organométallique,

ii) ou à acyler une benzylamine (VII) de formule

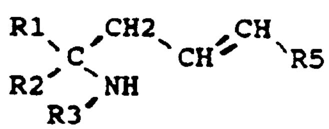

dans laquelle R3 est alkyle inférieur par un halogénure d'acyle R4-[(CH2)m-1]COZ4 dans lequel Z4 est le chlore ou le brome, pour obtenir un N-carboxamide intermédiaire (VIII)

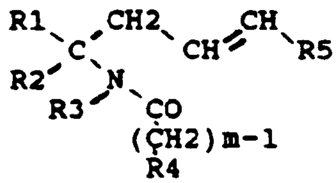

que l'on réduit par un hydrure métallique

iii) ou à faire réagir un réactif organomagnésien R2MgZ3 dans lequel Z3 est le chlore, le brome ou l'iode,sur un aminonitrile (IX) dans lequel R3 est alkyle inférieur.

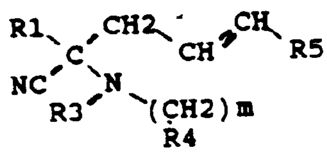

iiii) ou alkyler une benzylamine (VII) déjà décrite par un halogénure d'alkyle R'-(CH2)m-Z2 déjà décrit,

- pour obtenir un sel à salifier une benzylamine (I) par un acide et pour obtenir une forme optiquement active à résoudre un racémique.

**2.** Procédé suivant la revendication 1, caractérisé en ce que R1 est phényle.

**3.** Procédé suivant les revendications 1 ou 2, caractérisé en ce que R5 est phényle.

**4.** Procédé suivant les revendications 1 à 3, cacaractérisé en ce que R2 est éthyle.

**5.** Procédé suivant les revendications 1 à 4, caractérisé en ce que R3 est l'hydrogène ou méthyle.

**6.** Procédé suivant l'une des revendications 1 à 5, caractérisé en ce que Rx est un radical méthyle ou phényle.

**7.** Procédé de préparation d'un médicament, notamment psychotrope et utile aux traitements des psychoses, caractérisé en ce qu'il consiste à mélanger une N-cycloalkylalkyl benzylamine définie à l'une des revendications 1 à 6 à un excipient pharmaceutique.

**8.** Procédé de préparation d'un médicament utile au traitement des affections gastro-intestinales, caractérisé en ce qu'il consiste à mélanger une N-cycloalkylalkyl benzylamine définie à l'une des revendications 1 à 6 à un excipient pharmaceutique.

**9.** Composés intermédiaires de formule :

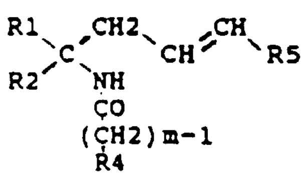

IV

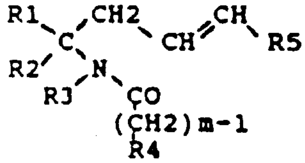

V III

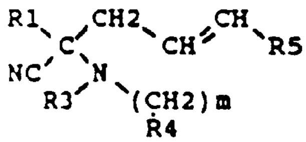

IX

dans lesquels R1, R2, R3, R4, R5 et m ont les significations désignées pour les benzylamines (I), R3 n'étant toutefois pas l'hydrogène.

10. L'utilisation d'une benzylamine telle que définie aux revendications 1 à 6 ou de l'un de ses sels thérapeutiquement administrables pour l'obtention d'un médicament psychotrope et utile aux traitements des psychoses.

11. L'utilisation d'une benzylamine telle que définie aux revendications 1 à 6 ou de l'un de ses sels thérapeutiquement administrables pour l'obtention d'un médicament utile en traitement des affections gastro-intestinales.

12. L'utilisation d'un composé intermédiaire tel que défini à la revendication 9 comme intermédiaire de synthèse de benzylamines.

## Patentansprüche

### Patentansprüche für folgende Vertragsstaaten : AT, BE, CH, DE, FR, GB, IT, LI, LU, NL, SE

1. Disubstituierte N-Cycloalkylalkyl-Benzylamine der nachstehenden Formel (I)

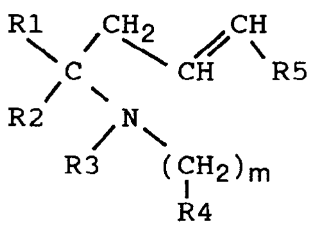

(I)

wobei
. R1 steht für einen Phenylrest, der gegebenenfalls mono-, di- oder trisubstituiert ist mit Halogenatomen, mit niederen Alkylresten, mit niederen halogenierten Alkylresten oder mit niederen Alkoxyresten;
. R2 steht für einen niederen Alkylrest;
. R3 steht für Wasserstoff oder für einen niederen Alkylrest;
. R4 steht für eine Cycloalkylgruppe - $CH(CH_2)_n$, wobei n einen ganzzahligen Wert von "2" bis "5" hat und wobei ein R4-C-Atom mit einem Rest Rx verknüpft sein kann, der für einen niederen Alkylrest oder für den Phenylrest steht;
. R5 steht für einen Phenylrest, der gegebenenfalls mono-, di- oder trisubstituiert ist mit Halogenatomen oder mit niederen Alkoxyresten;
. m einen Wert von "1 " oder "2" hat; einschließlich deren Additionssalze mit Säuren und deren optisch aktive Formen.

2. N-Cycloalkylalkyl-Benzylamine nach Anspruch 1,
<u>dadurch gekennzeichnet,</u> daß
der Rest R1 ein Phenylrest ist.

3. N-Cycloalkylalkyl-Benzylamine nach Anspruch 1 oder 2,
<u>dadurch gekennzeichnet,</u> daß
der Rest R5 ein Phenylrest ist.

4. N-Cycloalkylalkyl-Benzylamine nach einem der Ansprüche 1 bis 3,

dadurch gekennzeichnet, daß der Rest R2 ein Ethylrest ist.

**5.** N-Cycloalkylalkyl-Benzylamine nach einem der Ansprüche 1 bis 4, dadurch gekennzeichnet, daß der Rest R3 für Wasserstoff oder Methyl steht.

**6.** N-Cycloalkylalkyl-Benzylamine nach einem der Ansprüche 1 bis 5, dadurch gekennzeichnet, daß der Rest Rx für einen Methylrest oder Phenylrest steht.

**7.** Verfahren zur Herstellung von N-Cycloalkylalkyl-Benzylaminen entsprechend nachstehender Formel (I)

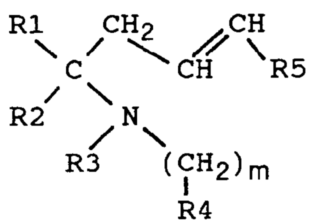

(I)

gekennzeichnet durch nachstehende alternative Verfahrensschritte:

. zur Erzeugung eines Benzylamin der Formel (II), welche der Formel (I) entspricht, wobei der Rest R3 für Wasserstoff steht:

i) wird ein Benzylamin der nachstehenden Formel (V)

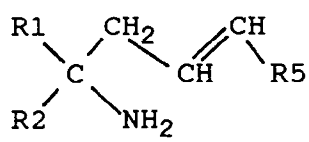

(V)

mit einem Acylierungsmittel (VI) der Formel

$(R4\text{-}[(CH_2)_{m-1}]\text{-}CO)_pZ1$ acyliert,

wobei Z1 für einen Hydroxylrest oder für Chlor oder Brom steht, wenn p den Wert "1 " hat, oder für ein Sauerstoffatom steht, wenn p den Wert "2" hat,

um eine N-Carboxamid-Zwischenstufe der nachstehenden Formel (IV) zu erhalten:

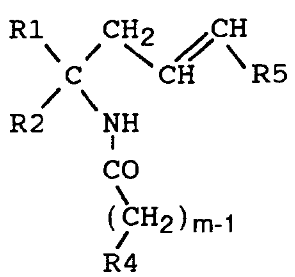

(IV)

und diese N-Carboxamid-Zwischenstufe wird mit einem Metallhydrid reduziert;

ii) wird ein Benzylamin der vorstehenden Formel (V) mit einem Cycloalkyalkylhalogenid der Formel $R4\text{-}(CH_2)_m\text{-}Z2$ alkyliert, wobei Z2 für Chlor, Brom oder Jod steht;

. zur Erzeugung eines Benzylamin der Formel (III), welche der Formel (I) entspricht, wobei der Rest R3 für einen niederen Alkylrest steht

i) wird ein Benzylamin (I) der Formel (II) unter reduzierenden Bedingungen mit einem Aldehyd R6-CHO alkyliert, wobei R6 für das kleinere C-Homologe zum Rest R3 steht (d.h. R3 = CH2 - R6)

wobei als Reduktionsmittel ein Metallhydrid oder ein Organometallhydrid eingesetzt wird;

ii) wird ein Benzylamin der nachstehenden Formel (VII)

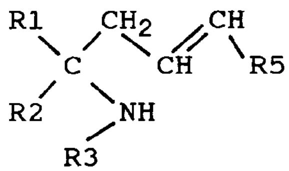

(VII)

wobei R3 für einen niederen Alkylrest steht, mit einem Acylhalogenid der Formel R4-($(CH_2)_{m-1}$]-COZ4 acyliert,
wobei Z4 für Chlor oder Brom steht,
um eine N-Carboxamid-Zwischenstufe der nachstehenden Formel (VIII) zu erhalten:

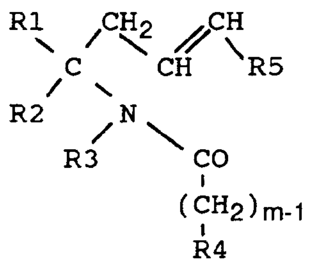

(VIII)

und diese N-Carboxamid-Zwischenstufe wird mit einem Metallhydrid reduziert;
iii) wird ein Organomagnesium-Reagens R2MgZ3, wobei Z3 für Chlor, Brom oder Jod steht, mit einem Aminonitril der nachstehenden Formel (IX) umgesetzt,

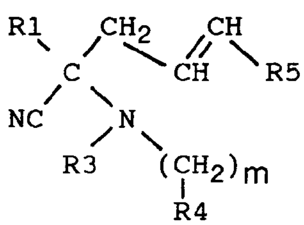

(IX)

wobei R3 für einen niederen Alkylrest steht.
iiii) wird ein Benzylamin der vorstehenden Formel (VII) mit einem Alkylhalogenid der Formel R'-$(CH_2)_m$-Z2 alkyliert,
wobei Z2 die vorstehend angegebene Bedeutung hat,
. zur Erzeugung eines Salzes erfolgt eine Salzbildung eines Benzylamin (I) mit einer Säure, und eine optisch aktive Form wird durch Trennung eines Racemates erhalten.

**8.** Arzneimittel, insbesondere mit psychotroper Wirkung und mit einer vorteilhaften Wirkung bei der Behandlung von Psychosen,
dadurch gekennzeichnet, daß
das Arzneimittel als Wirkstoff ein N-Cycloalkylalkyl-Benzylamin nach einem der Ansprüche 1 bis 6 enthält.

**9.** Arzneimittel mit vorteilhafter Wirkung bei Magen-Darm-Störungen,
dadurch gekennzeichnet, daß
das Arzneimittel als Wirkstoff ein N-Cycloalkylalkyl-Benzylamin nach einem der Ansprüche 1 bis 6 enthält.

**10.** Zwischenprodukte entsprechend nachstehender Formel:

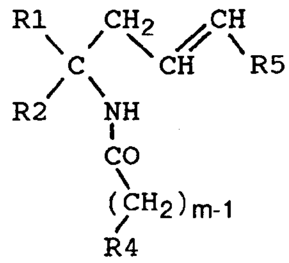

(IV)

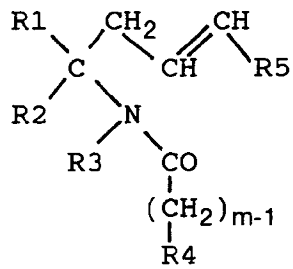

(VIII)

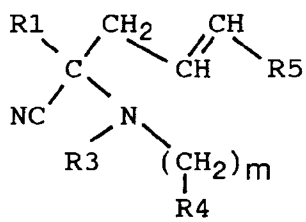

(IX)

wobei die Reste R1, R2, R3, R4, R5 und m die in Anspruch 1 für das N-Cycloalkylalkyl-Benzylamin (I) angegebene Bedeutung haben, wobei R3 jedoch nicht Wasserstoff ist.

**Patentansprüche für folgenden Vertragsstaat : ES**

1. Verfahren zur Herstellung von N-Cycloalkylalkyl-Benzylaminen entsprechend nachstehender Formel (I)

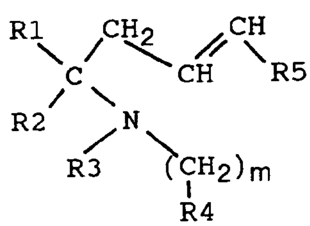

(I)

wobei

. R1 steht für einen Phenylrest, der gegebenenfalls mono-, di- oder trisubstituiert ist mit Halogenatomen, mit niederen Alkylresten, mit niederen halogenierten Alkylresten oder mit niederen Alkoxyresten;

. R2 steht für einen niederen Alkylrest;

. R3 steht für Wasserstoff oder für einen niederen Alkylrest;

. R4 steht für eine Cycloalkylgruppe - $CH(CH_2)_n$, wobei n einen ganzzahligen Wert von "2" bis "5" hat und wobei ein R4-C-Atom mit einem Rest Rx verknüpft sein kann, der für einen niederen Alkylrest oder für den Phenylrest steht;

. R5 steht für einen Phenylrest, der gegebenenfalls mono-, di- oder trisubstituiert ist mit Halogenatomen oder mit niederen Alkoxyresten;

. m einen Wert von "1 " oder "2" hat; einschließlich deren Additionssalze mit Säuren und deren optisch aktive Formen.

<u>gekennzeichnet durch</u> nachstehende alternative Verfahrensschritte:

. zur Erzeugung eines Benzylamin der Formel (II), welche der Formel (I) entspricht, wobei der Rest

R3 für Wasserstoff steht:
i) wird ein Benzylamin der nachstehenden Formel (V)

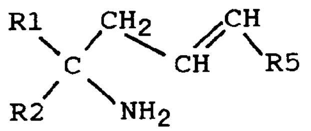

(V)

mit einem Acylierungsmittel (VI) der Formel
$(R4\text{-}[(CH_2)_{m-1}]\text{-}CO)_pZ1$ acyliert,
wobei Z1 für einen Hydroxylrest oder für Chlor oder Brom steht, wenn p den Wert "1 " hat, oder für ein Sauerstoffatom steht, wenn p den Wert "2" hat,
um eine N-Carboxamid-Zwischenstufe der nachstehenden Formel (IV) zu erhalten:

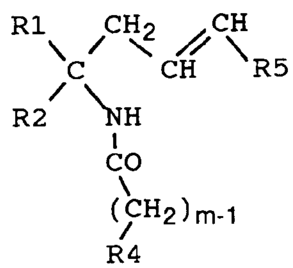

(IV)

und diese N-Carboxamid-Zwischenstufe wird mit einem Metallhydrid reduziert;
ii) wird ein Benzylamin der vorstehenden Formel (V) mit einem Cycloalkyalkylhalogenid der Formel $R4\text{-}(CH_2)_m\text{-}Z2$ alkyliert, wobei Z2 für Chlor, Brom oder Jod steht
. zur Erzeugung eines Benzylamin der Formel (III), welche der Formel (I) entspricht, wobei der Rest R3 für einen niederen Alkylrest steht
i) wird ein Benzylamin (I) der Formel (II) unter reduzierenden Bedingungen mit einem Aldehyd R6-CHO alkyliert,
wobei R6 für das kleinere C-Homologe zum Rest R3 steht (d.h. R3 = CH2 - R6)
wobei als Reduktionsmittel ein Metallhydrid oder ein Organometallhydrid eingesetzt wird;
ii) wird ein Benzylamin der nachstehenden Formel (VII)

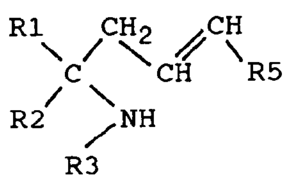

(VII)

wobei R3 für einen niederen Alkylrest steht, mit einem Acylhalogenid der Formel $R4\text{-}[(CH_2)_{m-1}]\text{-}COZ4$ acyliert,
wobei Z4 für Chlor oder Brom steht,
um eine N-Carboxamid-Zwischenstufe der nachstehenden Formel (VIII) zu erhalten:

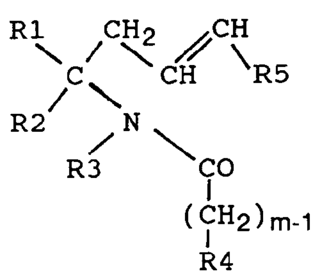

(VIII)

und diese N-Carboxamid-Zwischenstufe wird mit einem Metallhydrid reduziert;
iii) wird ein Organomagnesium-Reagens R2MgZ3, wobei Z3 für Chlor, Brom oder Jod steht, mit einem Aminonitril der nachstehenden Formel (IX) umgesetzt,

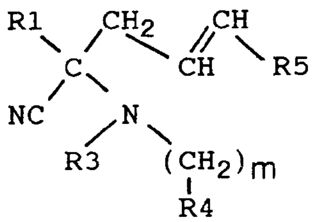

(IX)

wobei R3 für einen niederen Alkylrest steht.
iiii) wird ein Benzylamin der vorstehenden Formel (VII) mit einem Alkylhalogenid der Formel R'-$(CH_2)_m$-Z2 alkyliert,
wobei Z2 die vorstehend angegebene Bedeutung hat,
. zur Erzeugung eines Salzes erfolgt eine Salzbildung eines Benzylamin (I) mit einer Säure, und eine optisch aktive Form wird durch Trennung eines Racemates erhalten.

**2.** Verfahren nach Anspruch 1,
dadurch gekennzeichnet, daß
der Rest R1 ein Phenylrest ist.

**3.** Verfahren nach einem der Ansprüche 1 oder 2,
dadurch gekennzeichnet, daß
der Rest R5 ein Phenylrest ist.

**4.** Verfahren nach einem der Ansprüche 1 bis 3,
dadurch gekennzeichnet, daß
der Rest R2 ein Ethylrest ist.

**5.** Verfahren nach einem der Ansprüche 1 bis 4,
dadurch gekennzeichnet, daß
der Rest R3 für Wasserstoff oder Methyl steht.

**6.** Verfahren nach einem der Ansprüche 1 bis 5,
dadurch gekennzeichnet, daß
der Rest Rx für einen Methylrest oder Phenylrest steht.

**7.** Verfahren zur Herstellung eines Arzneimittels, insbesondere mit psychotroper Wirkung und mit einer vorteilhaften Wirkung bei der Behandlung von Psychosen,
dadurch gekennzeichnet, daß
ein N-Cycloalkylalkyl-Benzylamin nach einem der Ansprüche 1 bis 6 mit einem pharmazeutischen Träger vermischt wird.

**8.** Verfahren zur Herstellung eines Arzneimittels mit vorteilhafter Wirkung bei Magen-Darm-Störungen,
dadurch gekennzeichnet, daß
ein N-Cycloalkylalkyl-Benzylamin nach einem der Ansprüche 1 bis 6 mit einem pharmazeutischen Träger vermischt wird.

**9.** Zwischenprodukte nachstehender Formel:

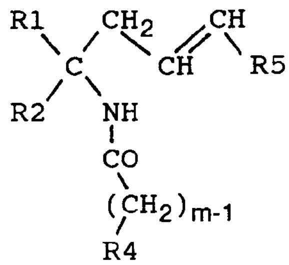

(IV)

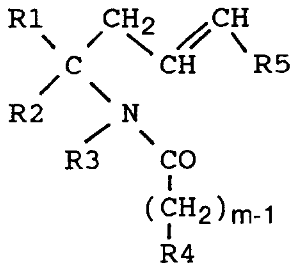

(VIII)

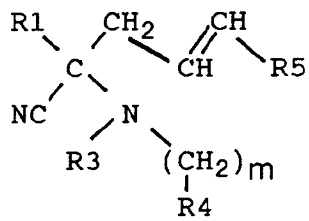

(IX)

wobei die Reste R1, R2, R3, R4, R5 und m die in Anspruch 1 für das N-Cycloalkylalkyl-Benzylamin (I) angegebene Bedeutung haben, wobei R3 jedoch nicht Wasserstoff ist.

**10.** Verwendung eines Benzylamin nach einem der Ansprüche 1 bis 6, oder eines seiner therapeutisch verträglichen Salze zur Erzeugung eines Arzneimittels mit vorteilhafter psychotroper Wirkung und mit einer vorteilhaften Wirkung bei der Behandlung von Psychosen.

**11.** Verwendung eines Benzylamin nach einem der Ansprüche 1 bis 6, zur Erzeugung eines Arzneimittels mit vorteilhafter Wirkung bei der Behandlung von Magen-Darm-Störungen.

**12.** Verwendung eines Zwischenproduktes, das nach Anspruch 9 erhältlich ist, als Zwischenprodukt zur Benzylaminsynthese.

**Patentansprüche für folgenden Vertragsstaat : GR**

**1.** Disubstituierte N-Cycloalkylalkyl-Benzylamine der nachstehenden Formel (I)

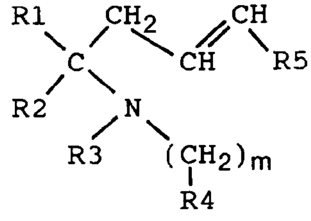

(I)

wobei

. R1 steht für einen Phenylrest, der gegebenenfalls mono-, di- oder trisubstituiert ist mit Halogenatomen, mit niederen Alkylresten, mit niederen halogenierten Alkylresten oder mit niederen Alkoxyresten;

. R2 steht für einen niederen Alkylrest;
. R3 steht für Wasserstoff oder für einen niederen Alkylrest;
. R4 steht für eine Cycloalkylgruppe - $CH(CH_2)_n$, wobei n einen ganzzahligen Wert von "2" bis "5" hat und wobei ein R4-C-Atom mit einem Rest Rx verknüpft sein kann, der für einen niederen Alkylrest oder für den Phenylrest steht;
. R5 steht für einen Phenylrest, der gegebenenfalls mono-, di- oder trisubstituiert ist mit Halogenatomen oder mit niederen Alkoxyresten;
. m einen Wert von "1 " oder "2" hat;
einschließlich deren Additionssalze mit Säuren und deren optisch aktive Formen.

**2.** N-Cycloalkylalkyl-Benzylamine nach Anspruch 1, <u>dadurch gekennzeichnet,</u> daß der Rest R1 ein Phenylrest ist.

**3.** N-Cycloalkylalkyl-Benzylamine nach Anspruch 1 oder 2, <u>dadurch gekennzeichnet,</u> daß der Rest R5 ein Phenylrest ist.

**4.** N-Cycloalkylalkyl-Benzylamine nach einem der Ansprüche 1 bis 3, <u>dadurch gekennzeichnet,</u> daß der Rest R2 ein Ethylrest ist.

**5.** N-Cycloalkylalkyl-Benzylamine nach einem der Ansprüche 1 bis 4, <u>dadurch gekennzeichnet,</u> daß der Rest R3 für Wasserstoff oder Methyl steht.

**6.** N-Cycloalkylalkyl-Benzylamine nach einem der Ansprüche 1 bis 5, <u>dadurch gekennzeichnet,</u> daß der Rest Rx für einen Methylrest oder Phenylrest steht.

**7.** Verfahren zur Herstellung von N-Cycloalkylalkyl-Benzylaminen entsprechend nachstehender Formel (I)

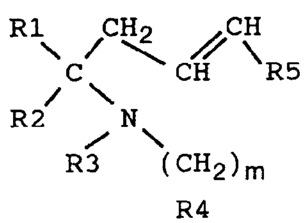

(I)

<u>gekennzeichnet durch</u> nachstehende alternative Verfahrensschritte:
. zur Erzeugung eines Benzylamin der Formel (II), welche der Formel (I) entspricht, wobei der Rest R3 für Wasserstoff steht:
i) wird ein Benzylamin der nachstehenden Formel (V)

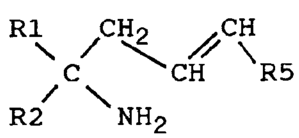

(V)

mit einem Acylierungsmittel (VI) der Formel
$(R4-[(CH_2)_{m-1}]-CO)_pZ1$ acyliert,
wobei Z1 für einen Hydroxylrest oder für ein Chlor- oder Bromatom steht, wenn p den Wert "1" hat, oder für ein Sauerstoffatom steht, wenn p den Wert "2" hat,
um eine N-Carboxamid-Zwischenstufe der nachstehenden Formel (IV) zu erhalten:

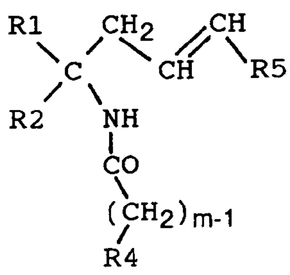

(IV)

und diese N-Carboxamid-Zwischenstufe wird mit einem Metallhydrid reduziert;

ii) wird ein Benzylamin der vorstehenden Formel (V) mit einem Cycloalkyalkylhalogenid der Formel R4-$(CH_2)_m$-Z2 alkyliert, wobei Z2 für Chlor, Brom oder Jod steht;

. zur Erzeugung eines Benzylamin der Formel (III), welche der Formel (I) entspricht, wobei der Rest R3 für einen niederen Alkylrest steht

i) wird ein Benzylamin (I) der Formel (II) unter reduzierenden Bedingungen mit einem Aldehyd R6-CHO alkyliert,

wobei R6 für das kleinere C-Homologe zum Rest R3 steht (d.h. R3 = $CH_2$ - R6)

wobei als Reduktionsmittel ein Metallhydrid oder ein Organometallhydrid eingesetzt wird;

ii) wird ein Benzylamin der nachstehenden Formel (VII)

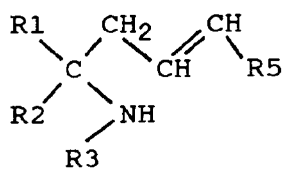

(VII)

wobei R3 für einen niederen Alkylrest steht, mit einem Acylhalogenid der Formel R4-[$(CH_2)_{m-1}$]-COZ4 acyliert,

wobei Z4 für Chlor oder Brom steht,

um eine N-Carboxamid-Zwischenstufe der nachstehenden Formel (VIII) zu erhalten:

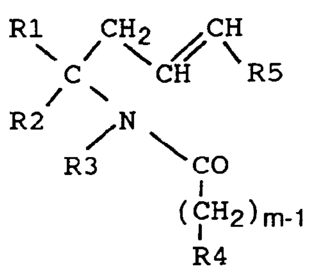

(VIII)

und diese N-Carboxamid-Zwischenstufe wird mit einem Metallhydrid reduziert;

iii) wird ein Organomagnesium-Reagens R2MgZ3, wobei Z3 für Chlor, Brom oder Jod steht, mit einem Aminonitril der nachstehenden Formel (IX) umgesetzt,

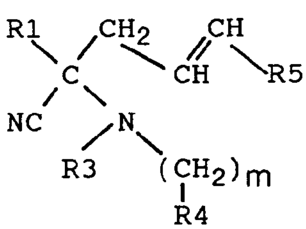

(IX)

wobei R3 für einen niederen Alkylrest steht.

iiii) wird ein Benzylamin der vorstehenden Formel (VII) mit einem Alkylhalogenid der Formel R'-$(CH_2)_m$-Z2 alkyliert,

wobei Z2 die vorstehend angegebene Bedeutung hat,
. zur Erzeugung eines Additionsalzes erfolgt eine Salzbildung eines Benzylamin (I) mit einer Säure, und eine optisch aktive Form wird durch Trennung eines Racemates erhalten.

8. Verfahren zur Herstellung eines Arzneimittels, insbesondere mit psychotroper Wirkung und mit einer vorteilhaften Wirkung bei der Behandlung von Psychosen,
dadurch gekennzeichnet, daß
ein N-Cycloalkylalkyl-Benzylamin nach einem der Ansprüche 1 bis 6 mit einem pharmazeutischen Träger vermischt wird.

9. Verfahren zur Herstellung eines Arzneimittels mit vorteilhafter Wirkung bei Magen-Darm-Störungen,
dadurch gekennzeichnet, daß
ein N-Cycloalkylalkyl-Benzylamin nach einem der Ansprüche 1 bis 6 mit einem pharmazeutischen Träger vermischt wird.

10. Verwendung eines Benzylamin nach einem der Ansprüche 1 bis 6, oder eines seiner therapeutisch verträglichen Salze zur Erzeugung eines Arzneimittels mit vorteilhafter psychotroper Wirkung und mit einer vorteilhaften Wirkung bei der Behandlung von Psychosen.

11. Verwendung eines Benzylamin nach einem der Ansprüche 1 bis 6, oder eines seiner therapeutisch verträglichen Salze zur Erzeugung eines Arzneimittels mit vorteilhafter Wirkung bei der Behandlung von Magen-Darm-Störungen.

12. Zwischenprodukte nachstehender Formel

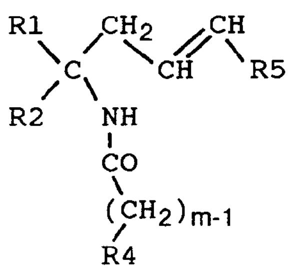

(IV)

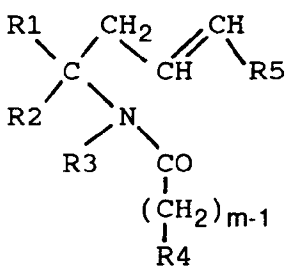

(VIII)

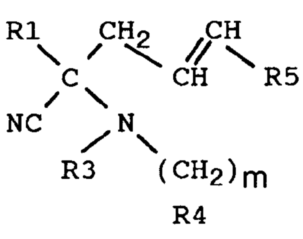

(IX)

wobei die Reste R1, R2, R3, R4, R5 und m die in Anspruch 1 für das N-Cycloalkylalkyl-Benzylamin (I) angegebene Bedeutung haben, wobei R3 jedoch nicht Wasserstoff ist.

## Claims

### Claims for the following Contracting States : AT, BE, CH, DE, FR, GB, IT, LI, LU, NL, SE

1. Disubstituted N-cycloalkylalkyl benzylamines of the formula I.

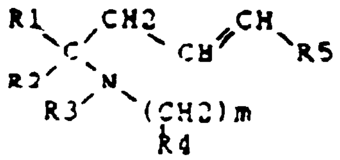

I

in which:
- R1 is phenyl possibly mono, di- or trisubstituted by halogen atoms, by lower alkyl radicals, by lower haloalkyl radicals or by lower alkoxy radicals;
- R2 is lower alkyl;
- R3 is hydrogen or lower alkyl;
- R4 is cycloalkyl - CH (CH2)n in which n has as its value an integer form 2 to 5, with an atom of carbon of R4 being able to bear a radical Rx which is lower alkyl or phenyl;
- R5 is phenyl possibly mono-, di- or trisubstituted by halogen atoms or by lower alkoxy radicals;
- m is 1 or 2;

their addition salts with acids and their optically active forms.

2. N-cycloalkylalkyl benzylamines as in claim 1 characterised in that R1 is phenyl.

3. N-cycloalkylalkyl benzylamines as in claims 1 or 2 characterised in that R5 is phenyl.

4. N-cycloalkylalkyl benzylamines as in claims 1 to 3 characterised in that R2 is ethyl.

5. N-cycloalkylalkyl benzylamines as in claims 1 to 4 characterised in that R3 is hydrogen or methyl.

6. N-cycloalkylalkyl benzylamines as in claims 1 to 5 characterised in that Rx is a methyl or phenyl radical.

7. Process for preparing N-cycloalkylalkyl benzylamines of formula (1) characterized in that it comprises:
- to obtain a benzylamine of formula (11) agreeing with (1) in which R3 is hydrogen:

i) acylating a benzylamine (V) of formula:

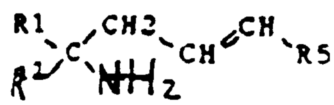

V

with an acylating agent (R4-(CH2)m-1) -CO)pZ1 (VI) in which Z1 is a hydroxyl radical or an atom of chlorine or bromine when p=1 or Z1 is an atom of oxygen when p=2, to obtain an intermediate N-carboxamide (1V)

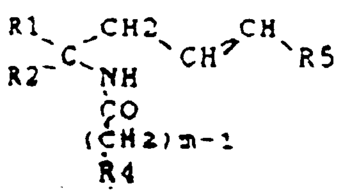

IV

and then reducing it with a metallic hydride

ii) alkylating a benzylamine (V) by a halide of cycloalkylalkyl R4-(CH2)m-Z2 in which Z2 is chlorine, bromine or iodine.
- to obtain a benzylamine of formula (III) agreeing with the formula (I) in which R3 is lower alkyl,

i) carrying out reductive alkylation of a benzylamine (1) of formula (11) of the invention with an aldehyde

R6-CHO in which R6 is the lower carbonaceous counterpart in the radical R3 (R3=CH2-R6) and a reducing agent such as a metallic or organometailic hydride,
ii) or acylating a benzylamine (VII) of the formula

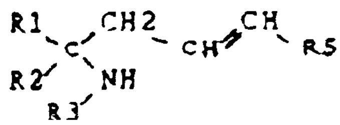

VII

in which R3 is lower alkyl with an acyl halide R4 - [(CH2)m-1] COZ4 in which Z4 is chlorine or bromine, in order to obtain an intermediate N-carboxamide (VIII).

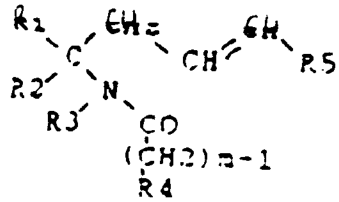

VIII

which is reduced with a metallic hydride
iii) or causing an organic magnesium reagent R2MgZ3, in which Z3 is chlorine, bromine or iodine, to react with an aminonitryl (IX) in which R3 is lower alkyl

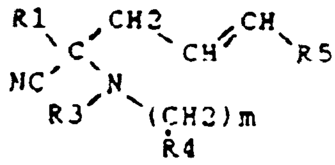

IX

iiii) or alkylating a benzylamine (VII) with an alkyl halide R - (CH2)m Z2 already described.
- to obtain an addition salt with an acid salifying the benzylamine with an acid and to obtain an optically active form resoluting a racemic.

**8.** Medicinal preparation, particularly psychotropic and of use in the treatments of psychoses, characterised in that it includes an N-cycloalkylalkyl benzylamine in accordance with one of claims 1 to 6.

**9.** Medicinal preparation of use in the treatment of gastro-intestinal complaints, characterised in that it includes an N-cycloalkylalkyl benzylamine in accordance with one of claims 1 to 6.

**10.** Intermediate compounds of the formula:

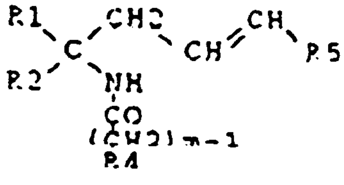

IV

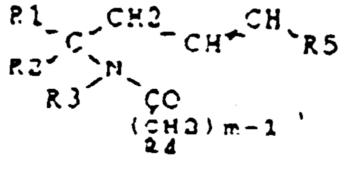

VIII

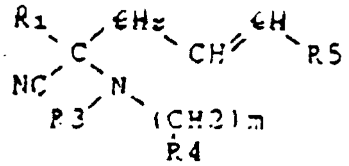

IX

in which R1, R2, R3, R4, R5 and m have the meanings defined for the benzylamines (1) but with R3 not being hydrogen.

**Claims for the following Contracting State : ES**

1. Process for preparing disubstituted N-cycloalkylalkyl benzylamines of the formula 1:

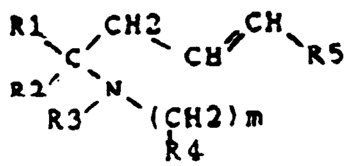

I

in which:
- R1 is phenyl possibly mono, di- or trisubstituted by halogen atoms, by lower alkyl radicals, by lower haloalkyl radicals or by lower alkoxy radicals;
- R2 is lower alkyl;
- R3 is hydrogen or lower alkyl;
- R4 is cycloalkyl - CH (CH2)n in which n has as its value an integer form 2 to 5, with an atom of carbon of R4 being able to bear a radical Rx which is lower alkyl or phenyl;
- R5 is phenyl possibly mono-, di- or trisubstituted by halogen atoms or by lower alkoxy radicals;
- m is 1 or 2;

their addition salts with acids and their optically active forms, characterised in that it comprises:
- to obtain a benzylamine of formula (11) agreeing with (1) in which R3 is hydrogen:

i) acylating a benzylamine (V) of formula:

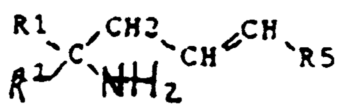

V

with an acylating agent (R4-(CH2)m-1)-CO)pZ1 (VI) in which Z1 is a hydroxyl radical or an atom of chlorine or bromine when p=1 or Z1 is an atom of oxygen when p=2, to obtain an intermediate N-carboxamide (1V)

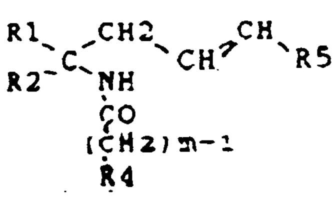

IV

and then reducing it with a metallic hydride

ii) alkylating a benzylamine (V) by a halide of cycloalkylalkyl R4-(CH2)m-Z2 in which Z2 is chlorine, bromine or iodine.
- to obtain a benzylamine of formula (III) agreeing with the formula (I) in which R3 is lower alkyl,

i) carrying out reductive alkylation of a benzylamine (1) of formula (11) of the invention with an aldehyde R6-CHO in which R6 is the lower carbonaceous counterpart in the radical R3 (R3=CH2-R6) and a reducing agent such as a metallic or organometailic hydride,

ii) or acylating a benzylamine (VII) of the formula

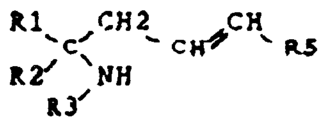

VII

in which R3 is lower alkyl with an acyl halide R4 - [(CH2)m-1] COZ4 in which Z4 is chlorine or bromine, in order to obtain an intermediate N-carboxamide (VIII).

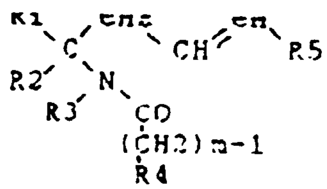

VIII

which is reduced with a metallic hydride
iii) or causing an organic magnesium reagent R2MgZ3, in which Z3 is chlorine, bromine or iodine, to react with an aminonitryl (IX) in which R3 is lower alkyl

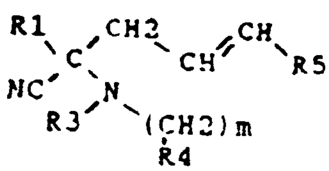

IX

iiii) or alkylating a benzylamine (VII) with an alkyl halide R - (CH2)m Z2 already described.
to obtain a salt salifying a benzylamine (I) with an acid and to obtain an optically active form resoluting a racemic.

**2.** Process as in claim 1 characterised in that R1 is phenyl.

**3.** Process as in claims 1 or 2 characterised in that R5 is phenyl.

**4.** Process as in claims 1 to 3 characterised in that R2 is ethyl.

**5.** Process as in claims 1 to 4 characterised in that R3 is hydrogen or methyl.

**6.** Process as in claims 1 to 5 characterised in that Rx is a methyl or phenyl radical.

**7.** Process for preparing a medicinal preparation, particularly psychotropic and of use in the treatments of psychoses, characterised in that it comprises mixing a N-cycloalkylalkyl benzylamine in accordance with one of claims 1 to 6 with a pharmaceutical carrier.

**8.** Process for preparing a medicinal preparation of use in treatment of gastro-intestinal complaints characterized in that it comprises mixing a N-cycloalkylalkyl benzylamine in accordance with one of claims 1 to 6 with a pharmaceutical carrier.

**9.** Intermediate compounds of the formula:

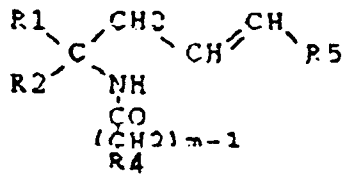

IV

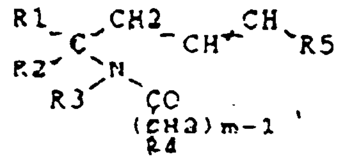

VIII

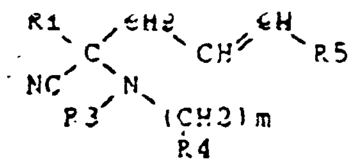

IX

in which R1, R2, R3, R4, R5 and m have the meanings defined for the benzylamines (1) but with R3 not being hydrogen.

10. The use of a benzylamine as defined in claims 1 to 6 or one of its therapeutically acceptable salts for preparing a psychotropic medicinal preparation and of use in the treatments of psychoses.

11. The use of a benzylamine as defined in claims 1 to 6 or one of its therapeutically acceptable salts for preparing a medicinal preparation of use in the treatment of gastro-intestinal complaints.

12. The use of an intermediate compound as defined in claim 9 as intermediate for synthesizing benzylamines.

**Claims for the following Contracting State : GR**

1. Disubstituted N-cycloalkylalkyl benzylamines of the formula 1.

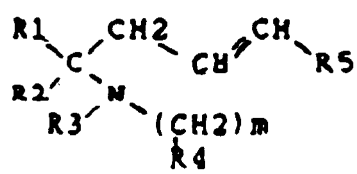

I

in which:
- R1 is phenyl possibly mono, di- or trisubstituted by halogen atoms, by lower alkyl radicals, by lower haloalkyl radicals or by lower alkoxy radicals;
- R2 is lower alkyl;
- R3 is hydrogen or lower alkyl;
- R4 is cycloalkyl - CH (CH2)n in which n has as its value an integer form 2 to 5, with an atom of carbon of R4 being able to bear a radical Rx which is lower alkyl or phenyl;
- R5 is phenyl possibly mono-, di- or trisubstituted by halogen atoms or by lower alkoxy radicals;
- m is 1 or 2;
their addition salts with acids and their optically active forms.

2. N-cycloalkylalkyl benzylamines as in claim 1 characterised in that R1 is phenyl.

3. N-cycloalkylalkyl benzylamines as in claims 1 or 2 characterised in that R5 is phenyl.

4. N-cycloalkylalkyl benzylamines as in claims 1 to 3 characterised in that R2 is ethyl.

5. N-cycloalkylalkyl benzylamines as in claims 1 to 4 characterised in that R3 is hydrogen or methyl.

6. N-cycloalkylalkyl benzylamines as in claims 1 to 5 characterised in that Rx is a methyl or phenyl radical.

7. Process for preparing N-cycloalkylalkyl benzylamines of formula (1) characterized in that it comprises:
- to obtain a benzylamine of formula (11) agreeing with (1) in which R3 is hydrogen:
i) acylating a benzylamine (V) of formula:

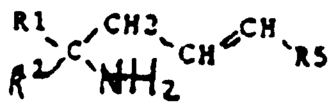

V

with an acylating agent (R4-(CH2)m-1) -CO)pZ1 (VI) in which Z1 is a hydroxyl radical or an atom of chlorine or bromine when p=1 or Z1 is an atom of oxygen when p=2, to obtain an intermediate N-carboxamide (1V)

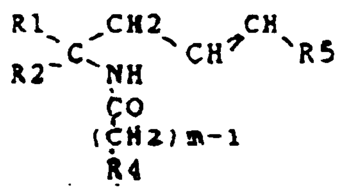

IV

and then reducing it with a metallic hydride
ii) alkylating a benzylamine (V) by a halide of cycloalkylalkyl R4-(CH2)m-Z2 in which Z2 is chlorine, bromine or iodine.
- to obtain a benzylamine of formula (III) agreeing with the formula (I) in which R3 is lower alkyl,
i) carrying out reductive alkylation of a benzylamine (1) of formula (11) of the invention with an aldehyde R6-CHO in which R6 is the lower carbonaceous counterpart in the radical R3 (R3=CH2-R6) and a reducing agent such as a metallic or organometailic hydride,
ii) or acylating a benzylamine (VII) of the formula

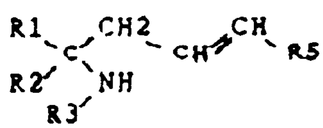

VII

in which R3 is lower alkyl with an acyl halide R4 - [(CH2)m-1] COZ4 in which Z4 is chlorine or bromine, in order to obtain an intermediate N-carboxamide (VIII).

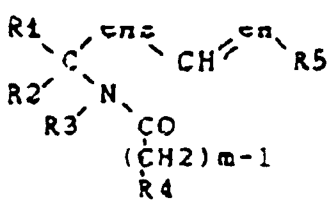

VIII

which is reduced with a metallic hydride
iii) or causing an organic magnesium reagent R2MgZ3, in which Z3 is chlorine, bromine or iodine, to react with an aminonitryl (IX) in which R3 is lower alkyl

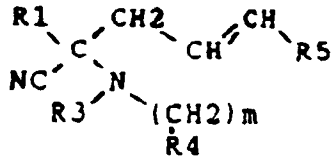

IX

iiii) or alkylating a benzylamine (VII) with an alkyl halide R - (CH2)m Z2 already described.

to obtain an addition salt with an acid salolifying the benzylamine with an acid and to obtain an optically active form resoluting a racemic.

**8.** Process for preparing a medicinal preparation, particularly psychotropic and of use in the treatments of psychoses, characterised in that it comprises mixing a N-cycloalkylalkyl benzylamine in accordance with one of claims 1 to 6 with a pharmaceutical carrier.

**9.** Process for preparing a medicinal preparation of use in the treatment of gastro- intestinal complaints, characterised in that it comprises mixing N-cycloalkylalkyl benzylamine in accordance with one of claims 1 to 6 with a pharmaceutical carrier.

**10.** The use of a benzylamine as defined in claims 1 to 6 or one of its therapeutically acceptable salts for preparing a psychotropic medicinal preparation or a medicinal preparation of use in the treatment of psychoses.

**11.** The use of a benzylamine as defined in claims 1 to 6 or one of its therapeutically acceptable salts for preparing a medicinal preparation of use in the treatment of gastro-intestinal complaints.

**12.** Intermediate compounds of formula

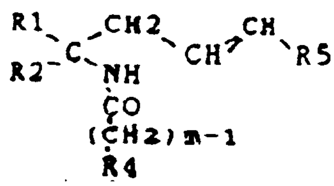

IV

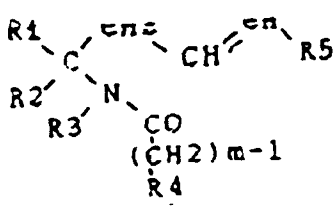

VIII

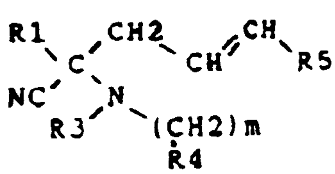

IX

in which R1, R2, R3, R4 and R5 have the meanings defined for the benzylamines (I) but with R3 not being hydrogen.